# EUROPEAN PATENT APPLICATION

(11) **EP 4 534 091 A1**
(43) Date of publication of application: **09.04.2025**
(21) Application number: 23816167.3
(22) Date of filing: 02.06.2023
(51) Int. Cl.: A61K 31/7088, A61K 38/02, A61K 39/395, A61K 48/00, A61P 9/00, A61P 9/04, A61P 39/00, A61P 43/00, C12Q 1/48, C12Q 1/68, G01N 33/15, G01N 33/50, G01N 33/53, A61K 31/165, A61K 31/704, A61K 45/06, A61P 35/00

(54) **INHIBITORY AGENT FOR MYOCARDIAL CELL DEATH, AND PROPHYLACTIC OR THERAPEUTIC AGENT FOR MYOCARDIAL DISORDERS OR HEART FAILURE**

(30) Priority: 02.06.2022 JP 2022090047; 10.08.2022 JP 2022127778
(71) Applicant: KYOTO PREFECTURAL PUBLIC UNIVERSITY CORPORATION, Kyoto-shi, Kyoto 602-8566 (JP)
(72) Inventor: KITANI, Tomoya, Kyoto-shi, Kyoto 602-8566 (JP); MATOBA, Satoaki, Kyoto-shi, Kyoto 602-8566 (JP)
(74) Representative: Winter, Brandl - Partnerschaft mbB
(86) International application number: PCT/JP2023/020619
(87) International publication number: WO 2023/234410

(57) **Abstract**

An object is to provide an inhibitory agent for myocardial cell death and a prophylactic or therapeutic agent for myocardial disorders or heart failure. The object described above is solved by the inhibitory agent for the myocardial cell death or the prophylactic or therapeutic agent for the myocardial disorders or the heart failure, the inhibitory agent or the prophylactic or therapeutic agent containing at least one component selected from the group consisting of a TAOK1 function inhibitor and a TAOK1 expression inhibitor.

## Description

### Technical Field

The present invention relates to an inhibitory agent for myocardial cell death, a prophylactic or therapeutic agent for myocardial disorders or heart failure, and the like.

### Background Art

Adriamycin (doxorubicin) is being widely used as a therapeutic agent for leukemia, breast cancer, and the like. However, since the adriamycin often causes cardiotoxicity (myocardial disorders or heart failure), continuation of a treatment with the adriamycin may be limited. In particular, it has been reported that an old age, a history of a cardiovascular disease, an experience of a radiation therapy, an experience of a concomitant chemotherapy, and the like increase a frequency of onset of the cardiotoxicity.

For the cardiotoxicity of the adriamycin, only a dexrazoxane has been approved by the US FDA as a therapeutic agent. However, the dexrazoxane has a carcinogenic effect and may attenuate an anticancer effect of the adriamycin.

TAOK1 is a serine-threonine protein kinase, and is involved in regulation of a stress-responsive MAP kinase pathway including a p38 and an extracellular signal-regulated protein kinase (ERK) kinase (MEKs). PTL 1 describes that a TAOK1 inhibitor may have an anticancer effect.

### Citation List

### Patent Literature

PTL 1: JP 2007-527412 A

### Summary of Invention

### Technical Problem

An object of the present invention is to provide an inhibitory agent for myocardial cell death and a prophylactic or therapeutic agent for myocardial disorders or heart failure.

### Solution to Problem

As a result of intensive studies in view of the above problems, the present inventor has found that the above problems can be solved by using at least one component selected from the group consisting of a TAOK1 function inhibitor and a TAOK1 expression inhibitor. As a result of further studies based on this finding, the present inventor has completed the present invention. In other words, the present invention includes the following aspects.

Item 1. An inhibitory agent for myocardial cell death, containing at least one component selected from the group consisting of a TAOK1 function inhibitor and a TAOK1 expression inhibitor.

Item 1A. A method for suppressing myocardial cell death, including administering, to a subject, at least one component selected from the group consisting of a TAOK1 function inhibitor and a TAOK1 expression inhibitor.

Item 1B. An agent containing at least one component selected from the group consisting of a TAOK1 function inhibitor and a TAOK1 expression inhibitor, the agent being use in suppressing myocardial cell death.

Item 1C. Use of at least one component selected from the group consisting of a TAOK1 function inhibitor and a TAOK1 expression inhibitor for producing an inhibitory agent for myocardial cell death.

Item 1D. Use of at least one component selected from the group consisting of a TAOK1 function inhibitor and a TAOK1 expression inhibitor for suppressing myocardial cell death.

Item 2. The inhibitory agent according to Item 1, in which the component is at least one selected from the group consisting of a polynucleotide targeting TAOK1, an expression cassette of the polynucleotide, a low molecular weight compound, a peptide, a protein, and an antibody.

Item 3. The inhibitory agent according to Item 1, in which the myocardial cell death is myocardial cell death caused by an anticancer agent treatment.

Item 4. The inhibitory agent according to Item 1, used to be administered in combination with an anticancer agent.

Item 5. The inhibitory agent according to Item 3 or 4, in which the anticancer agent is an anthracycline-based anticancer agent.

Item 6. The inhibitory agent according to Item 1, for use in prevention or treatment of a heart disease.

Item 7. The inhibitory agent according to Item 6, in which the heart disease is at least one heart disease selected from the group consisting of a myocardial disorder and heart failure.

Item 8. The inhibitory agent according to Item 6, in which the heart disease is a heart disease caused by an anticancer agent treatment.

Item 9. A prophylactic or therapeutic agent for at least one heart disease selected from the group consisting of a myocardial disorder and heart failure, the prophylactic or therapeutic agent containing at least one component selected from the group consisting of a TAOK1 function inhibitor and a TAOK1 expression inhibitor.

Item 9A. A method for preventing or treating a heart disease, comprising administering, to a subject, at least one component selected from the group consisting of a TAOK1 function inhibitor and a TAOK1 expression inhibitor.

Item 9B. An agent containing at least one component selected from the group consisting of a TAOK1 function inhibitor and a TAOK1 expression inhibitor, the agent being used in prevention or treatment of a heart disease.

Item 9C. Use of at least one component selected from the group consisting of a TAOK1 function inhibitor and a TAOK1 expression inhibitor for producing a prophylactic or therapeutic agent for a heart disease.

Item 9D. Use of at least one component selected from the group consisting of a TAOK1 function inhibitor and a TAOK1 expression inhibitor for prevention or treatment of a heart disease.

Item 10. The prophylactic or therapeutic agent according to Item 9, in which the heart disease is a heart disease caused by an anticancer agent treatment.

Item 11. A method for screening an active component of an inhibitory agent for myocardial cell death or an active component of a prophylactic or therapeutic agent for at least one heart disease selected from the group consisting of a myocardial disorder and heart failure, using, as an index, an amount, a concentration, or kinase activity of TAOK1 in a test sample derived from an animal or a cell treated with a test substance.

### Advantageous Effects of Invention

According to the present invention, it is possible to provide an inhibitory agent for myocardial cell death and a prophylactic or therapeutic agent for myocardial disorders or heart failure. Further, according to the present invention, it is also possible to provide a method for screening an active component of an inhibitory agent for myocardial cell death or an active component of a prophylactic or therapeutic agent for at least one heart disease selected from the group consisting of a myocardial disorder and heart failure.

### Brief Description of Drawings

Fig. 1 illustrates an outline of screening of genes involved in myocardial cell death with a doxorubicin (Test Example 1).
Fig. 2 illustrates viability of cardiomyocytes after a doxorubicin treatment (Test Example 2). The vertical axis indicates cell viability and the horizontal axis indicates doxorubicin concentrations. NTC KO indicates a case of using a non-target gRNA, TAOK1 KO indicates a case of using a gRNA targeting TAOK1, and 1 and 2 indicate cases of using different types of gRNAs.
Fig. 3 illustrates live cell/dead cell staining images of the cardiomyocytes after the doxorubicin treatment (Test Example 2).
Fig. 4 illustrates the viability of the cardiomyocytes after the doxorubicin treatment (Test Example 3). The vertical axis indicates cell viability, and in the horizontal axis, C43 10nM indicates a case of a treatment with a TAOK1 inhibitor (PTL 1: a compound 43) (final concentration: 10nM), and Ctrl indicates a negative control without a treatment with the TAOK1 inhibitor. The concentrations during the doxorubicin treatment are illustrated above the graph.
Fig. 5 illustrates an outline of Test Example 4.
Fig. 6 illustrates TAOK1 mRNA amount measurement results (left) and left ventricular ejection fractions (LVEF) (right) (Test Example 4). The horizontal axis indicates an elapsed period from administration of AAV9-sh-TAOK1 targeting TAOK1 or non-targeting AAV-sh-Scramble.
Fig. 7 illustrates measurement results of oxygen consumption (Test Example 5). In the legend, NTC KO indicates the case of using the non-target gRNA, and TAOK1 KO indicates the case of using the gRNA targeting TAOK1. The legend indicates doxorubicin treatment concentrations. The horizontal axis indicates an elapsed period of time from start of the test, and the vertical axis indicates oxygen consumption rates. Arrows in the graph indicate timings at which drugs illustrated above are added.
Fig. 8 illustrates measurement results of mitochondrial membrane potentials (left graph, vertical axis) and production amount of reactive oxygen species (right graph, vertical axis) (Test Example 6). The horizontal axis indicates the doxorubicin treatment concentrations (unit: µM).
Fig. 9 illustrates a proportion of γH2AX-positive cells in troponin T-positive cells (Test Example 7). The vertical axis indicates the proportion of the γH2AX-positive cells in the troponin T-positive cells. In the horizontal axis, NTC KO indicates the case of using the non-target gRNA, and TAOK1 KO indicates the case of using the gRNA targeting TAOK1. The horizontal axis indicates the doxorubicin treatment concentrations.
Fig. 10 illustrates measurement results of a proportion of an amount of a phosphorylated p38 to an amount of a p38 (Test Example 8). The vertical axis indicates the proportion of the amount of the phosphorylated p38 to the amount of the p38. In the horizontal axis, NTC KO indicates the case of using the non-target gRNA, TAOK1 KO indicates the case of using the gRNA targeting TAOK1, and DOX indicates a doxorubicin treatment group.
Fig. 11 illustrates measurement results of cell viability (Test Example 9). The vertical axis indicates the cell viability. The horizontal axis indicates cell lines used. The legend indicates siRNA used.
Fig. 12 illustrates tissue staining images (Test Example 10). The siRNA used is illustrated on the left sides of the photographic images. The type of staining is illustrated above the photographic images.
Fig. 13 illustrates cell staining images (Test Example 11). Presence or absence of a hypoxic load is illustrated on the left side of the photographic images. The siRNA used is illustrated above the photographic images.
Fig. 14 illustrates measurement result of a proportion of dead cells (Test Example 12). The vertical axis indicates the proportion of the dead cells. The horizontal axis indicates the siRNA used.

### Description of Embodiments

### 1. Definitions

In the present description, expressions "containing" and "including" include concepts of "containing", "including", "essentially consisting of", and "only consisting of".

"Identity" between amino acid sequences refers to a degree of amino acid sequence consistency between two or more comparable amino acid sequences. Accordingly, as the consistency between two amino acid sequences increases, the identity or similarity between those sequences increases. A level of the identity between the amino acid sequences is determined, for example, using FASTA, a tool for sequence analysis, and using default parameters. Alternatively, it can be determined using an algorithm BLAST (KarlinS, Altschul SF. "Methods for assessing the statistical significance of molecular sequence features by using general scoringschemes" Proc Natl Acad Sci USA. 87:2264-2268 (1990), KarlinS, Altschul SF. "Applications and statistics for multiple high-scoring segments in molecular sequences." Proc Natl Acad Sci USA. 90:5873-7 (1993)) by Karlin and Altschul. A program called BLASTX based on such BLAST algorithm has been developed. Specific procedures of these analysis methods are publicly known, and can be found on the National Center of Biotechnology Information (NCBI) website (http://www.ncbi.nlm.nih.gov/). In addition, "identity" between base sequences is also defined in accordance with the above.

In the description, "conservative substitution" means that an amino acid residue is substituted with an amino acid residue having a similar side chain. For example, substitution between amino acid residues having basic side chains such as lysine, arginine, or histidine corresponds to the conservative substitution. In addition, the conservative substitution also corresponds to substitution between amino acid residues having acidic side chains, such as an aspartic acid or a glutamic acid; amino acid residues having non-charged polar side chains, such as glycine, asparagine, glutamine, serine, threonine, tyrosine, and cysteine; amino acid residues having non-polar side chains, such as alanine, valine, leucine, isoleucine, proline, phenylalanine, methionine, or tryptophan; amino acid residues having a β-branched side chains, such as threonine, valine, or isoleucine; and amino acid residues having aromatic side chains, such as tyrosine, phenylalanine, tryptophan, or histidine.

In the present description, "nucleic acid" and "polynucleotide" are not particularly limited, and include both natural and artificial ones. Specifically, in addition to a DNA, an RNA, and the like, one subjected to a publicly known chemical modification may be possible as an example below. In order to prevent degradation by a hydrolase such as a nuclease, a phosphate residue (phosphate) of each of nucleotides can be substituted with a chemically modified phosphate residue such as phosphorothioate (PS), methyl phosphonate, or phosphorodithionate, or the like. In addition, a hydroxyl group at a 2-position of a sugar (ribose) of each ribonucleotide may be substituted with -OR (R represents, for example, CH3 (2'-O-Me), CH2CH2OCH3 (2'-O-MOE), CH2CH2NHC(NH)NH2, CH2CONHCH3, CH2CH2CN, or the like). Further, a base moiety (pyrimidine or purine) may be subjected to a chemical modification, and examples of the chemical modification include introduction of a methyl group or a cationic functional group to a 5-position of a pyrimidine base, and substitution of a carbonyl group at a 2-position with thiocarbonyl. Further, examples thereof include ones in which a phosphoric acid moiety or a hydroxyl moiety is modified with, for example, biotin, an amino group, a lower alkylamine group, or an acetyl group, and are not limited thereto. In addition, it is also possible to use BNA (LNA) or the like in which conformation of a sugar moiety is fixed to N-type by crosslinking 2' oxygen and 4' carbon of the sugar moiety of each of the nucleotides.

### 2. Inhibitory Agent for Myocardial Cell Death, or Prophylactic or Therapeutic Agent for Myocardial Disorders or Heart Failure

In one aspect, the present invention relates to an inhibitory agent for myocardial cell death or a prophylactic or therapeutic agent for myocardial disorders or heart failure (also referred to in the present description as an "agent of the present invention"), the inhibitory agent or the prophylactic or therapeutic agent containing at least one component selected from the group consisting of a TAOK1 function inhibitor and a TAOK1 expression inhibitor. These will be described below.

### 2-1. Subject to be Suppressed (TAOK1)

A TAOK1 gene is a gene encoding the serine-threonine protein kinase. TAOK1 (a TAOK1 protein or a TAOK1 mRNA) subjected to expression inhibition or function inhibition is an expression product of the TAOK1 gene, and is the TAOK1 protein or the TAOK1 mRNA expressed in an organism to which the agent of the present invention is applied or cells thereof (particularly the cardiomyocytes). Accordingly, the TAOK1 protein and the TAOK1 mRNA to be suppressed also change in accordance with biological species of a subject. The biological species are not particularly limited, and examples thereof include various mammals such as animals, for example, humans, monkeys, mice, rats, dogs, cats, rabbits, pigs, horses, cows, sheep, goats, and deer.

Amino acid sequences of the TAOK1 protein and base sequences of the TAOK1 mRNA derived from various biological species are publicly known. Specifically, examples of a human TAOK1 protein include a protein having an amino acid sequence represented by SEQ ID NO: 1 (NCBI Reference Sequence: NP_065842.1) and a protein having an amino acid sequence represented by SEQ ID NO: 2 (NCBI Reference Sequence: NP_079418.1). Examples of a human TAOK1 mRNA include an mRNA having a base sequence represented by SEQ ID NO: 3 (NCBI Reference Sequence: NM_020791.4) and an mRNA having a base sequence represented by SEQ ID NO: 4 (NCBI Reference Sequence: NM_025142.1). In addition, as the TAOK1 protein and the TAOK1 mRNA, the above splicing variants can also be included.

The TAOK1 protein to be suppressed may have an amino acid mutation such as substitution, deletion, addition, or insertion as long as the TAOK1 protein retains inherent properties, that is, serine-threonine protein kinase activity. Examples of the mutation include preferably the substitution and more preferably the conservative substitution, from a viewpoint that the activity is less likely to be impaired.

The TAOK1 mRNA to be suppressed may also have a base mutation such as substitution, deletion, addition, or insertion as long as a protein translated from the mRNA retains inherent properties, that is, the serine-threonine protein kinase activity. The mutation is preferably a mutation in which no amino acid substitution occurs or a mutation in which conservative amino acid substitution occurs in the protein translated from the mRNA.

Preferred specific examples of the TAOK1 protein to be suppressed include a protein described in the following (a) and a protein described in the following (b):
(a) a protein having an amino acid sequence represented by SEQ ID NO: 1 or 2; and
(b) at least one protein selected from the group consisting of proteins having amino acid sequences having 85% or more of identity to the amino acid sequence represented by SEQ ID NO: 1 or 2 and having the serine-threonine protein kinase activity.

In the above (b), the identity is more preferably 90% or more, still more preferably 95% or more, and even more preferably 98% or more.

Examples of the protein described in the above (b) include:
(b') a protein having an amino acid sequence in which one or more amino acids have been substituted, deleted, added, or inserted with respect to the amino acid sequence represented by SEQ ID NO: 1 or 2, and having the serine-threonine protein kinase activity.

In the above (b'), "more than one" refers to, for example, 2 to 50, preferably 2 to 20, more preferably 2 to 10, and still more preferably 2 or 3.

Preferred specific examples of the TAOK1 mRNA to be suppressed include an mRNA described in the following (c) and an mRNA described in the following (d):
(c) an mRNA having the base sequence represented by SEQ ID NO: 3 or 4; and
(d) at least one mRNA selected from the group consisting of mRNAs having base sequences having 85% or more of identity to the base sequence represented by SEQ ID NO: 3 or 4 and encoding the proteins having the serine-threonine protein kinase activity.

In the above (d), the identity is more preferably 90% or more, still more preferably 95% or more, and even more preferably 98% or more.

Examples of the mRNA described in the above (d) include
(d') an mRNA having a base sequence in which one or more bases are substituted, deleted, added, or inserted with respect to the base sequence represented by SEQ ID NO: 3 or 4 and encoding proteins having the serine-threonine protein kinase activity.

In the above (d'), "more than one" refers to, for example, 2 to 150, preferably 2 to 60, more preferably 2 to 30, and still more preferably 2 to 10.

### 2-2. Active Components

The active components of the agent of the present invention are at least one component selected from the group consisting of the TAOK1 expression inhibitor and the TAOK1 function inhibitor. Examples of the component preferably include the polynucleotide targeting TAOK1, the expression cassette of the polynucleotide, the low molecular weight compound, the peptide, the protein, and the antibody. Hereinafter, the TAOK1 expression inhibitor and the TAOK1 function inhibitor will be specifically described.

### 2-2-1. TAOK1 Expression Inhibitor

The TAOK1 expression inhibitor is not particularly limited as long as the inhibitor can suppress expression levels of the TAOK1 protein and/or the TAOK1 mRNA expressed in the organism to which the agent of the present invention is applied or the cells thereof (particularly cells of a lung tissue). The TAOK1 expression inhibitor can be used singly or in combination of two or more of inhibitors.

Examples of the TAOK1 expression inhibitor include a TAOK1-specific small interfering RNA (siRNA), a TAOK1-specific microRNA (miRNA), a TAOK1-specific antisense nucleic acid, and expression cassettes thereof, as well as a TAOK1-specific ribozyme, and a TAOK1 gene editing agent used in a CRISPR/Cas system.

Note that the expression inhibition means that expression levels of the TAOK1 protein, the TAOK1 mRNA, and the like are suppressed to, for example, 1/2, 1/3, 1/5, 1/10, 1/20, 1/30, 1/50, 1/100, 1/200, 1/300, 1/500, 1/1000, or 1/10000 or less, and also includes that the expression levels are set to 0.

### 2-2-1-1. siRNA, miRNA, Antisense Nucleic Acid, and Ribozyme

The TAOK1-specific siRNA is not particularly limited as long as the siRNA is a double-stranded RNA molecule that specifically suppresses expression of a gene encoding TAOK1. In one embodiment, the siRNA preferably has a length of, for example, 18 bases or more, 19 bases or more, 20 bases or more, or 21 bases or more. In addition, the siRNA preferably has a length of, for example, 25 bases or less, 24 bases or less, 23 bases or less, or 22 bases or less. It is assumed that an upper limit value and a lower limit value of the length of the siRNA described herein can be arbitrarily combined. For example, there is assumed a combination in length of a lower limit of 18 bases and an upper limit of 25 bases, 24 bases, 23 bases, or 22 bases; a lower limit of 19 bases and an upper limit of 25 bases, 24 bases, 23 bases, or 22 bases; a lower limit of 20 bases and an upper limit of 25 bases, 24 bases, 23 bases, or 22 bases; or a lower limit of 21 bases and an upper limit of 25 bases, 24 bases, 23 bases, or 22 bases.

The siRNA may be a shRNA (small hairpin RNA). The shRNA can be designed to have a portion forming a stem loop structure. For example, if a sequence of a certain region is set as a sequence a and a complementary strand to the sequence a is set as a sequence b, the shRNA can be designed such that these sequences are present in one RNA strand in an order of the sequence a, a spacer, and the sequence b, and designed to have a length of 45 to 60 bases in total. The sequence a is a sequence of a partial region of a base sequence encoding the target TAOK1, and the target region is not particularly limited, and any region can be made a candidate. Then, a length of the sequence a is 19 to 25 bases, and preferably 19 to 21 bases.

The TAOK1-specific siRNA may have an additional base at the 5'- or 3'-end thereof. The length of the additional base is usually about 2 to 4 bases. The additional base may be a DNA or an RNA, and if DNA is used, the stability of the nucleic acid may be improved. Examples of a sequence of such additional base include, but are not limited to, sequences such as ug-3', uu-3', tg-3', tt-3', ggg-3', guuu-3', gttt-3', ttttt-3', and uuuuu-3'.

The siRNA may have a protruding sequence (overhang) at the 3'-end thereof, and specific examples thereof include one to which dTdT (dT represents deoxythymidine) is added. In addition, the siRNA may have a blunt end without terminal addition. The siRNA may have a sense strand and an antisense strand with different numbers of bases, and examples thereof include an "asymmetric interfering RNA (aiRNA)" in which the antisense strand has protruding sequences (overhangs) at the 3'-end and the 5'-end thereof. In a typical aiRNA, the antisense strand has 21 bases, the sense strand has 15 bases, and both ends of the antisense strand each have an overhang structure of 3 bases.

A position of a target sequence of the TAOK1-specific siRNA is not particularly limited, and in one embodiment, it is desirable to select a target sequence from 5'-UTR, a region from a start codon to about 50 bases, and a region other than 3'-UTR. For a candidate group of the selected target sequence, it is preferable to examine whether or not there is a homology in consecutive 16-17 base sequences in the mRNA other than the target using homology search software such as BLAST (http://www.ncbi.nlm.nih.gov/BLAST/) to confirm the specificity of the selected target sequence. As for the target sequence of which specificity has been confirmed, the siRNA may be designed to be a double-stranded RNA having a sense strand with a 3' terminal overhang of TT or UU at 19-21 bases after AA (or NA), and an antisense strand having a complementary sequence to the 19-21 bases and the 3' terminal overhang of TT or UU. In addition, the shRNA that is a precursor of the siRNA can be designed by appropriately selecting any linker sequence (for example, about 5-25 bases) capable of forming a loop structure and linking the sense strand and the antisense strand via the linker sequence.

Sequences of the siRNA and/or the shRNA can be searched using search software provided on various websites for free. Examples of such site include the followings. siRNA Target Finder (http://www.ambion.com/jp/techlib/misc/siRNA_finder.html) and pSilencer (registered trademark) Expression Vector insert design tool (http://www.ambion.com/jp/techlib/misc/psilencer_converter.html) provided by Ambion, as well as GeneSeer (http://codex.cshl.edu/scripts/newsearchhairpin.cgi) provided by RNAi Codex.

The siRNA can be prepared by synthesizing a sense strand and an antisense strand of a target sequence on the mRNA with a DNA/RNA automatic synthesizer, respectively, denaturing the sense strand and the antisense strand at about 90°C° to about 95°C for about 1 minute in an appropriate annealing buffer, and then annealing the sense strand and the antisense strand at about 30°C to about 70°C for about 1 to about 8 hours. In addition, the siRNA can also be prepared by synthesizing the shRNA to be the precursor of the siRNA and cleaving the shRNA using an RNA cleaving protein dicer.

The TAOK1-specific miRNA is optional as long as the miRNA inhibits translation of the gene encoding TAOK1. For example, instead of cleaving a target mRNA as cleaving the siRNA, the miRNA may be paired with a 3' untranslated region (UTR) of the target to inhibit the translation. The miRNA may be any of a primary miRNA (pri-miRNA), a precursor miRNA (pre-miRNA), and a mature miRNA. The length of the miRNA is not particularly limited, and a length of the pri-miRNA is usually several hundred to several thousand bases, a length of the pre-miRNA is usually 50 to 80 bases, and a length of the mature miRNA is usually 18 to 30 bases. In one embodiment, the TAOK1-specific miRNA is preferably the pre-miRNA or the mature miRNA, and more preferably the mature miRNA. Such TAOK1-specific miRNA may be synthesized by a publicly known method or may be purchased from a company that provides the synthetic RNA.

The TAOK1-specific antisense nucleic acid refers to a nucleic acid containing a base sequence complementary or substantially complementary to a base sequence of an mRNA of the gene encoding TAOK1 or a part thereof, and a nucleic acid having a function of suppressing TAOK1 protein synthesis by forming and binding a specific and stable double-stranded chain with the mRNA. The antisense nucleic acid may be any of a DNA, an RNA, and a DNA/RNA chimera. In a case where the antisense nucleic acid is the DNA, an RNA:DNA hybrid formed by a target RNA and an antisense DNA is recognized by an endogenous ribonuclease H (RNase H) and causes selective degradation of the target RNA. Accordingly, in a case of the antisense DNA directed to the degradation by the RNase H, the target sequence may be not only a sequence in the mRNA but also a sequence of an intron region in an initial translation product of the TAOK1 gene. An intron sequence can be determined by comparing a genomic sequence with a cDNA base sequence of the TAOK1 gene using a homology search program such as BLAST or FASTA.

The length of a target region of the TAOK1-specific antisense nucleic acid is not limited as long as the antisense nucleic acid is hybridized to inhibit translation into the TAOK1 protein as a result. The TAOK1-specific antisense nucleic acid may be the entire sequence or a partial sequence of the mRNA encoding TAOK1. An oligonucleotide having about 10 to about 40 bases, particularly about 15 to about 30 bases is preferable in consideration of simplification of synthesis, problems of antigenicity and intracellular migration, and the like, and is not limited thereto. More specifically, a 5'-end hairpin loop, a 5'-end untranslated region, a translation start codon, a protein coding region, an ORF translation-terminating codon, a 3'-end untranslated region, a 3'-end palindromic region, a 3'-end hairpin loop, or the like of the TAOK1 gene can be selected as a preferred target region of the antisense nucleic acid, and the target region is not limited thereto.

The TAOK1-specific antisense nucleic acid may be one (antigene) that not only can be hybridized with the mRNA or an initial transcript of the TAOK1 gene to inhibit the translation into the protein, but also can bind to these genes that are double-stranded DNAs to form a triple-stranded chain (triplex) and inhibit transcription into RNA.

The TAOK1-specific siRNA, the TAOK1-specific miRNA, the TAOK1-specific antisense nucleic acid, and the like can be prepared by determining a target sequence of the mRNA or the initial transcript based on a cDNA sequence or a genomic DNA sequence of the TAOK1 gene, and synthesizing a sequence complementary thereto using a commercially available DNA/RNA automated synthesizer. In addition, antisense nucleic acids containing various modifications each can also be chemically synthesized by a publicly known method.

The expression cassette of the TAOK1-specific siRNA, the TAOK1-specific miRNA, or the TAOK1-specific antisense nucleic acid is not particularly limited as long as the expression cassette is a polynucleotide in which the TAOK1-specific siRNA, the TAOK1-specific miRNA, or the TAOK1-specific antisense nucleic acid is incorporated in an expressible state. Typically, the expression cassette includes a promoter sequence and a polynucleotide having a coding sequence for the TAOK1-specific siRNA, the TAOK1-specific miRNA, or the TAOK1-specific antisense nucleic acid (if necessary, further a transcription-terminating signal sequence), and optionally other sequences. The promoter is not particularly limited, and examples thereof include an RNA polymerase II (polII)-based promoter such as a CMV promoter, an EF1 promoter, an SV40 promoter, an MSCV promoter, an hTERT promoter, a β-actin promoter, or a CAG promoter; and an RNA polymerase III (polIII)-based promoter such as mouse and human U6-snRNA promoters, a human H1-RNase P RNA promoter, or a human valine-tRNA promoter. Among these promoters, the polIII-based promoter is preferable from a viewpoint that transcription of a short RNA can be accurately performed. In addition, various promoters that can be induced by a drug can also be used. The other sequences are not particularly limited, and various publicly known sequences that can be contained in an expression vector can be adopted. Examples of such sequences include a replication origin and a drug resistance gene. In addition, examples of the type of the drug resistance gene and the type of the vector are as described above.

Another example of the TAOK1 expression inhibitor includes a TAOK1-specific ribozyme. The "ribozyme" in a narrow sense means an RNA having enzyme activity of cleaving a nucleic acid, and in the present application, also includes a DNA as long as the ribozyme has sequence-specific nucleic acid cleaving activity. A most versatile ribozyme nucleic acid is a self-splicing RNA found in an infectious RNA such as viroid and virusoid, and known as hammerhead-type or hairpin-type ribozyme nucleic acid. The hammerhead type ribozyme nucleic acid exerts enzyme activity at about 40 bases, and can specifically cleave only a target mRNA by making each of several bases at both ends adjacent to a portion having a hammerhead structure (about 10 bases in total) into a sequence complementary to a desired cleavage site of the mRNA. This type of ribozyme nucleic acid has an advantage that the ribozyme nucleic acid does not attack a genomic DNA because only the RNA is used as a substrate. In a case where the mRNA of the TAOK1 gene itself has a double-stranded structure, the target sequence can be made single-stranded by using a hybrid ribozyme in which an RNA motif derived from a viral nucleic acid capable of specifically binding to an RNA helicase is linked [Proc. Natl. Acad. Sci. USA, 98(10): 5572-5577 (2001)]. Further, in a case where the ribozyme is used in a form of an expression vector containing a DNA encoding the ribozyme, a hybrid ribozyme in which a tRNA-modified sequence is further linked can also be used in order to promote translocation of a transcript to cytoplasm [Nucleic Acids Res., 29(13): 2780-2788 (2001)].

### 2-2-1-2. Gene Editing Agent

The TAOK1 gene editing agent is not particularly limited as long as the agent can suppress the expression of the TAOK1 gene by a target sequence-specific nuclease system (for example, the CRISPR/Cas system). The expression inhibition of the TAOK1 gene can be achieved by, for example, disruption of the TAOK1 gene or suppression of the activity of a promoter by modification of the promoter of the TAOK1 gene.

As the TAOK1 gene editing agent, for example, in a case of adopting the CRISPR/Cas system, typically, it is possible to use a vector (a TAOK1 gene editing vector) including a guide RNA expression cassette targeting the TAOK1 gene or the promoter thereof and a Cas protein expression cassette, and the TAOK1 gene editing agent is not limited thereto. In addition to this typical example, for example, it is possible to use a combination of the vector including the guide RNA targeting the TAOK1 gene or the promoter thereof and/or the expression cassette thereof and a vector including a Cas protein and/or an expression cassette thereof as the TAOK1 gene editing agent.

The guide RNA expression cassette is not particularly limited as long as the expression cassette is a polynucleotide used for the purpose of expressing the guide RNA in a target organism. Typical examples of the expression cassette include a promoter and a polynucleotide having the entire or partial coding sequence for the guide RNA arranged under control of the promoter. Note that, in other words, "arranged under control of the promoter" means that the guide RNA-coding sequence is arranged such that transcription of the sequence is controlled by the promoter. Examples of a specific arrangement mode include a mode in which the guide RNA-coding sequence is arranged immediately below a 3'-side of the promoter (for example, a mode in which the number of base pairs (bp) between a base at the 3'-end of the promoter and a base at the 5'-end of the guide RNA-coding sequence is, for example, 100 bp or less, and preferably 50 bp or less).

The promoter of the guide RNA expression cassette is not particularly limited, and the pol II-based promoter can also be used. However, the pol III-based promoter is preferable from a viewpoint of more accurately performing transcription of a relatively short RNA. The pol III-based promoter is not particularly limited, and examples thereof include the mouse and human U6-snRNA promoters, the human H1-RNase P RNA promoter, and the human valine-tRNA promoter. In addition, various promoters that can be induced by a drug can also be used.

The guide RNA-coding sequence is not particularly limited as long as the coding sequence is a base sequence encoding the guide RNA.

The guide RNA is not particularly limited as long as the guide RNA can be used in the CRISPR/Cas system, and for example, it is possible to use various guide RNAs capable of inducing the Cas protein to a target site of the genomic DNA by binding to the target site of the genomic DNA (for example, the TAOK1 gene, the promoter thereof, or the like) and binding to the Cas protein.

In the present description, the target site is a site on the genomic DNA including a DNA strand (a target strand) having a proto-spacer adjacent motif (PAM) sequence and a sequence of about 17 to 30 bases in length (preferably 18 to 25 bases in length, more preferably 19 to 22 bases in length, and particularly preferably 20 bases in length) adjacent to the 5' side thereof, and a complementary DNA strand (a non-target strand).

The PAM sequence varies depending on the type of the Cas protein utilized. For example, the PAM sequence corresponding to a Cas9 protein (type II) derived from S. pyogenes is 5'-NGG, the PAM sequence corresponding to a Cas9 protein (type I-A1) derived from S. solfataricus is 5'-CCN, the PAM sequence corresponding to a Cas9 protein (type I-A2) derived from S. solfataricus is 5'-TCN, the PAM sequence corresponding to a Cas9 protein (type I-B) derived from H. walsbyl is 5'-TTC, the PAM sequence corresponding to a Cas9 protein (type I-E) derived from E. coli is 5'-AWG, the PAM sequence corresponding to a Cas9 protein (type I-F) derived from E. coli is 5'-CC, the PAM sequence corresponding to a Cas9 protein (type I-F) derived from P. aeruginosa is 5'-CC, the PAM sequence corresponding to a Cas9 protein (type II-A) derived from S. Thermophilus is 5'-NNAGAA, the PAM sequence corresponding to a Cas9 protein (type II-A) derived from S. agalactiae is 5'-NGG, the PAM sequence corresponding to a Cas9 protein derived from S. aureus is 5'-NGRRT or 5'-NGRRN, the PAM sequence corresponding to a Cas9 protein derived from N. meningitidis is 5'-NNNNGATT, and the PAM sequence corresponding to a Cas9 protein derived from T. denticola is 5'-NAAAAC.

The guide RNA has a sequence (also referred to as a crRNA (CRISPR RNA) sequence) involved in binding to the target site of the genomic DNA, and this crRNA sequence complementarily (preferably, complementarily and specifically) binds to a sequence excluding a sequence complementary to the PAM sequence of the non-target strand, whereby the guide RNA can bind to the target site of the genomic DNA.

Note that the "complementary" binding includes not only a case of binding based on a complete complementary relationship (A and T as well as G and C) but also a case of binding based on a complementary relationship to the extent that hybridization can be performed under stringent conditions. The stringent conditions can be determined on the basis of a melting temperature (Tm) of the nucleic acid binding a complex or a probe as taught by Berger and Kimmel (1987, Guide to Molecular Cloning Techniques Methods in Enzymology, Vol. 152, Academic Press, San Diego CA). For example, examples of washing conditions after the hybridization usually include conditions of about "1 x SSC, 0.1% SDS, 37°C". A hybridization state is preferably maintained even when the washing is performed under such conditions. Although not particularly limited, examples of more stringent hybridization conditions include washing conditions of about "0.5 x SSC, 0.1% SDS, 42°C" and examples of even more stringent hybridization conditions include washing conditions of about "0.1 x SSC, 0.1% SDS, 65°C".

Specifically, among the crRNA sequences, the sequence that binds to the target sequence has, for example, 90% or more, preferably 95% or more, more preferably 98% or more, still more preferably 99% or more, and particularly preferably 100% identity to the target strand. Note that it is said that 12 bases on the 3'-side of the sequence binding to the target sequence among the crRNA sequences are important for binding of the guide RNA to the target site. Therefore, in a case where the sequence binding to the target sequence among the crRNA sequences is not completely identical to the target strand, the bases different from the target strand are preferably present in a portion other than the 12 bases on the 3'-side of the sequence binding to the target sequence among the crRNA sequences.

The guide RNA has a sequence (also referred to as a trans-activating crRNA (tracrRNA) sequence) involved in the binding to the Cas protein, and this tracrRNA sequence binds to the Cas protein, whereby the Cas protein can be induced at the target site of the genomic DNA.

The tracrRNA sequence is not particularly limited. The tracrRNA sequence is typically an RNA having a sequence of about 50 to 100 bases in length capable of forming a plurality of (usually 3) stem loops, and the sequence varies depending on the type of the Cas protein utilized. As the tracrRNA sequence, various publicly known sequences can be adopted depending on the type of the Cas protein utilized.

The guide RNA usually includes the crRNA sequence and the tracrRNA sequence described above. The form of the guide RNA may be a single-stranded RNA (sgRNA) having the crRNA sequence and the tracrRNA sequence, or may be an RNA complex in which the RNA having the crRNA sequence and the RNA having the tracrRNA sequence are complementarily bound.

The Cas protein expression cassette is not particularly limited as long as the expression cassette is a polynucleotide used for the purpose of expressing the Cas protein in a target organism. Typical examples of the expression cassette include a promoter and a polynucleotide having a Cas protein-coding sequence arranged under control of the promoter. Note that "arranged under control of the promoter" is similar to the definition in the guide RNA expression cassette.

The promoter of the Cas protein expression cassette is not particularly limited, and for example, various pol II-based promoters can be used. Examples of the pol II-based promoters include, but are not limited to, for example for example, the CMV promoter, the EF1 promoter, the SV40 promoter, the MSCV promoter, the hTERT promoter, the β-actin promoter, and the CAG promoter. In addition, various promoters that can be induced by a drug can also be used.

The Cas protein-coding sequence is not particularly limited as long as the coding sequence is a base sequence encoding an amino acid sequence of the Cas protein.

The Cas protein is not particularly limited as long as the protein can be used in the CRISPR/Cas system, and for example, it is possible to use various Cas proteins capable of binding to the target site of the genomic DNA in a state where a complex with the guide RNA has been formed and cleaving the target site. Cas proteins derived from various organisms have been known, and examples thereof include the Cas9 protein (type II) derived from S. pyogenes, the Cas9 protein (type I-A1) derived from S. solfataricus, the Cas9 protein (type I-A2) derived from S. solfataricus, the Cas9 protein (type I-B) derived from H. walsbyl, the Cas9 protein (type I-E) derived from E. coli, the Cas9 protein (type I-F) derived from E. coli, the Cas9 protein (type I-F) derived from P. aeruginosa, the Cas9 protein (type II-A) derived from S. Thermophilus, the Cas9 protein (type II-A) derived from S. agalactiae, the Cas9 protein derived from S. aureus, the Cas9 protein derived from N. meningitidis, the Cas9 protein derived from T. denticola, and the Cpf1 protein (type V) derived from F. novicida. Among the Cas proteins, the Cas9 protein is preferable, and a Cas9 protein endogenously possessed by a bacterium belonging to genus Streptococcus is more preferable. Amino acid sequences of various Cas proteins and information on the coding sequences thereof can be easily obtained on various databases such as NCBI.

The Cas protein may be a wild-type double-stranded truncated Cas protein or a nickase-type Cas protein. In addition, as long as the activity of the Cas protein is not impaired, the Cas protein may have a mutation (for example, substitution, deletion, insertion, or addition) in the amino acid sequence, or may be added with a publicly known a protein tag, a signal sequence, or an enzyme protein. Examples of the protein tag include biotin, a His tag, a FLAG tag, a Halo tag, an MBP tag, an HA tag, a Myc tag, a V5 tag, and a PA tag. Examples of the signal sequence include a cytoplasmic translocation signal.

The TAOK1 gene editing vector may have other sequences. The other sequences are not particularly limited, and various publicly known sequences that can be contained in an expression vector can be adopted. Examples of such sequences include a replication origin and a drug resistance gene.

The TAOK1 gene editing agent can be easily produced in accordance with a publicly known genetic engineering technique. For example, the editing agent can be produced utilizing PCR, restriction enzyme cleavage, a DNA linkage technique, an in vitro transcription/translation technique, a recombinant protein production technique, or the like.

### 2-2-2. TAOK1 Function Inhibitor

The TAOK1 function inhibitor is not particularly limited as long as the inhibitor can regulate the functions of the TAOK1 protein and/or the TAOK1 mRNA expressed in the organism to which the agent of the present invention is applied or the cells thereof (particularly the cells of the lung tissue). The TAOK1 function inhibitor can be used singly or in combination of two or more of inhibitors.

The TAOK1 function inhibitor is not particularly limited as long as the inhibitor can reduce the serine-threonine protein kinase activity.

Examples of the TAOK1 function inhibitor include a low molecular weight compound (for example, having a molecular weight of 1000 or less, 800 or less, 700 or less, 600 or less, or 500 or less; or a molecular weight of 100 or more, 150 or more, or 200 or more).

A large number of low molecular weight compounds having a TAOK1 function inhibitory action have been reported in various literatures. For example, in PTL 1: J Enzyme Inhib Med Chem. 2021; 36(1): 98-108., Molecular cancer therapeutics vol. 16,11 (2017): 2410-2421. Acta neuropathologica communications vol. 6,1 37. 7 May. 2018 and the like, a low molecular weight compound having a TAOK1 function inhibitory action has been reported. Specific examples of such low molecular weight compound are as follows.

**Table 1**

| Entry | Name | Structure |
|---|---|---|
| | N-[2-oxo-2-(1,2,3,4-tetrahydronaphthalene-1-ylamino)ethyl]-4-(pentyloxy)benzamide | 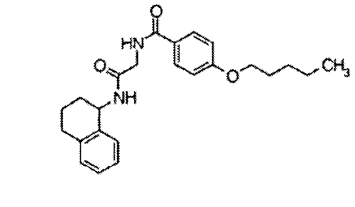 |
| 2 | N~2~-{({4-[(phenylmethyl)oxy]phenyl}oxy) acetyl]-N-(1,2,3,4-tetrahydronaphthalene-1-yl)glycinamide | 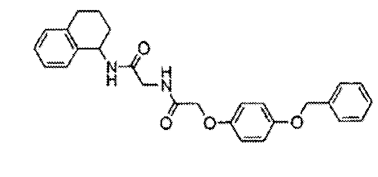 |
| 3 | N~2~-{[(4-bromophenyl)oxy]acetyl}-N-(1,2,3,4-tetrahydronaphthalen-1-yl)glycinamide | 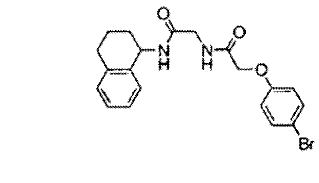 |
| 4 | 4'-ethyl-N-[2-oxo-2-(1,2,3,4-tetrahydronaphthalen-1-ylamino)ethyl]biphenyl 4-carboxamide | 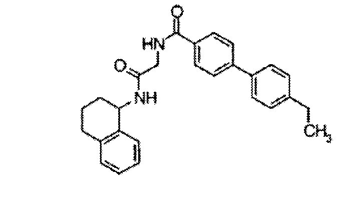 |
| 5 | 4'-ethyl-N-[1-methyl-2-oxo-2-(1,2,3,4-tetrahydronaphthalen-2-ylamino)ethyl]biphenyl 4-carboxamide | 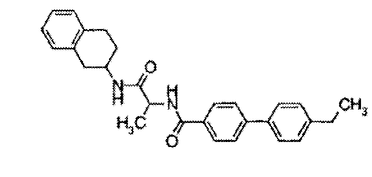 |

**Table 2**

| Entry | Name | Structure |
|---|---|---|
| 6 | 4-(hexyloxy)-N-[2-oxo-2-(1,2,3,4-tetrahydronaphthalen-1-ylamino)ethyl]benzamide | 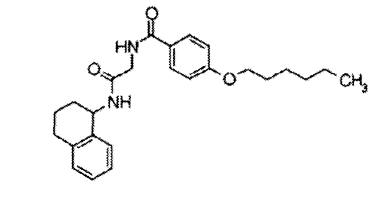 |
| 7 | 2-cyclopentyl-N-[2-oxo-1-pyridin-3-yl-2-(1,2,3,4-tetrahydronaphthalen-1-ylamino)ethyl]-2-phenylacetamide | 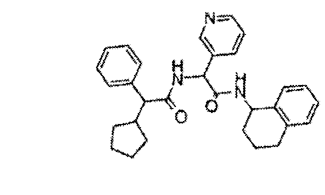 |
| 8 | 4-(heptyloxy)-N-[2-oxo-2-(1,2,3,4-tetrahydronaphthalen-1-ylamino)ethyl]benzamide | 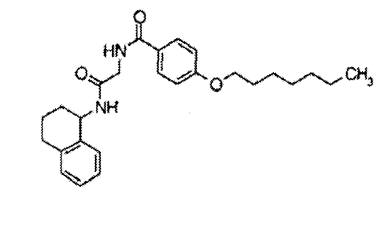 |
| 9 | N-[1-methyl-2-oxo-2-(1,2,3,4-tetrahydronaphthalen-2-ylamino)ethyl]-4-(pentyloxy)benzamide | 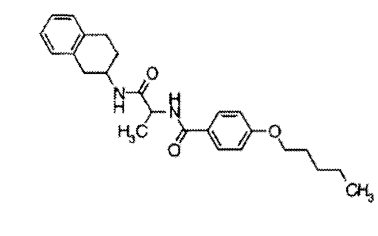 |
| 10 | 4-hexyl-N-[2-oxo-2-(1,2,3,4-tetrahydronaphthalen-1-ylamino)ethyl]benzamide | 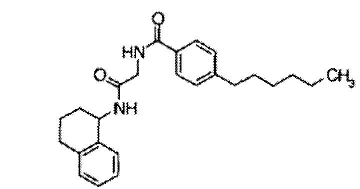 |

**Table 3**

| Entry | Name | Structure |
|---|---|---|
| 11 | N-[2-oxo-2-(1,2,3,4-tetrahydronaphthalen-1-ylamino)ethyl]-4-pentylbenzamide | 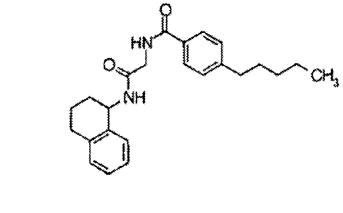 |
| 12 | 4-heptyl-N-[2-oxo-2-(1,2,3,4-tetrahydronaphthalen-1-ylamino)ethyl]benzamide | 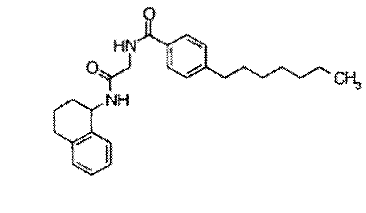 |
| 13 | Na-{[5,6-bis(methyloxy)-1H-indol-2-yl]carbonyl}-N-(1,2,3,4-tetrahydronaphthalen-1-yl)tryptophanamide | 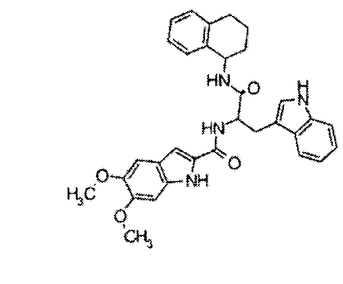 |
| 14 | Na-{[4-(butyloxy)phenyl]carbonyl}-N-{1,2,3,4-tetrahydronaphthalen-1-yl)tryptophanamide | 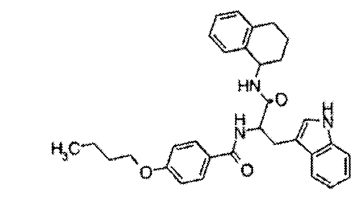 |

**Table 4**

| Entry | Name | Structure |
|---|---|---|
| 15 | 5-{(2E)-3-[3,4-bis(methyloxy)phenyl]brob-2-enoyl}-N-[2-(3-chlorophenyl)ethyl]-4,5,6,7-tetrahydro-3H-imidazo[4,5-c]pyridine-6-carboxamide | 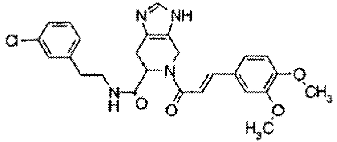 |
| 16 | N-[2-oxo-1-pyridin-3-yl-2-(1,2,3,4-tetrahydronaphthalen-1-ylamino)ethyl]-4-(1H-1,2,4-triazol-1-yl)benzamide | 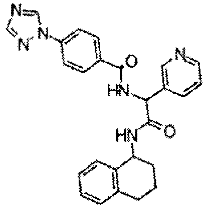 |
| 17 | Na-{(2E)-3-[3,4-bis(methyloxy)phenyl]brob-2-enoyl}-N-(1,2,3,4-tetrahydronaphthalen-1-yl)tryptophanamide | 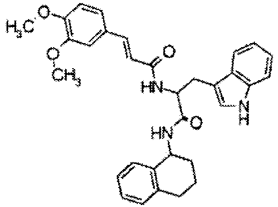 |
| 18 | 4-(butyloxy)-N-[1-methyl-2-oxo-2-(1,2,3,4-tetrahydronaphthalen-2-ylamino)ethyl]benzamide | 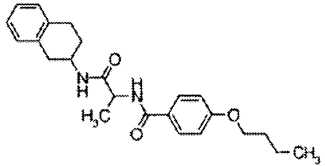 |
| 19 | N-[1-methyl-2-oxo-2-(1,2,3,4-tetrahydronaphthalen-2-ylamino)ethyl]-4-[(phenylmethyl)oxy]benzamide | 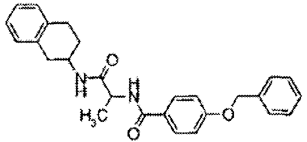 |

**Table 5**

| Entry | Name | Structure |
|---|---|---|
| 20 | 3,5-dimethyl-N-[2-oxo-2-(1,2,3,4-tetrahydronaphthalen-1-ylamino)ethyl]-4-[(phenylmethyl)oxy]benzamide | 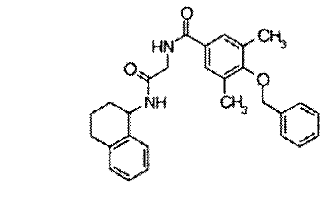 |
| 21 | N~2~-({[4-(butyloxy)phenyl]amino}carbon yl)-N-(2-phenylethyl)-O-(phenylmethyl)serinamide | 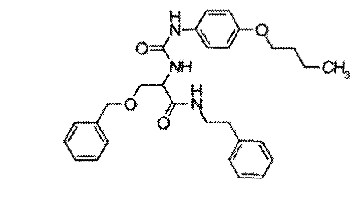 |
| 22 | (2S)-N-1 ~-(4-butylphenyl)-N~2~-[3,4-dichlorophenyl)methyl]pyrrolidin e-1,2-dicarboxamide | 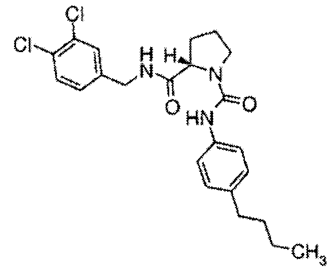 |
| 23 | N-[6-(methyloxy)-1,3-benzothiazol-2-yl]-4-{[4-oxo-5,6,7,8-tetrahydro[1]benzothieno[2,3-d]pyrimidin-3(4H)-yl)acetyl]amino}butanamide | 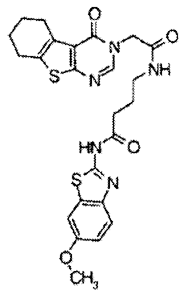 |

**Table 6**

| Entry | Name | Structure |
|---|---|---|
| 24 | (2S)-N~2~-[(3,4-dichlorophenyl)methyl]-N~1~-[4-(1-methylethyl)phenyl]pyrrolidine-1,2-dicarboxamide | 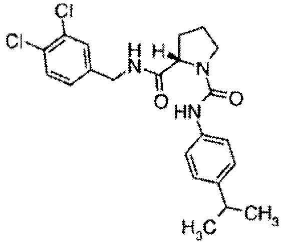 |
| 25 | N~2~-([(4-bromophenyl)oxy]acetyl)-N-[(3,4-dichlorophenyl)methyl]glycinam ide | 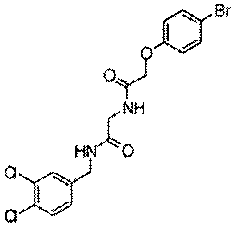 |
| 26 | N-[2-oxo-1-pyridin-3-yl-2-(1,2,3,4-tetrahydronaphthalen-1-ylamino)ethyl]-4-[(E)-phenyldiazenyl]benzamide | 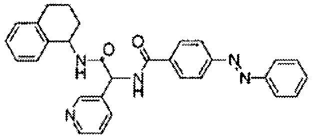 |
| 27 | 4-(butyloxy)-N-[2-oxo-2-(1,2,3,4-tetrahydronaphthalen-1-ylamino)ethyl]benzamide | 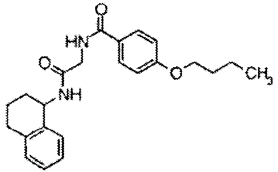 |
| 28 | N~2~-({[3-(methyloxy)phenyl]oxy}acetyl)-N-(1,2,3,4-tetrahydronaphthalen-1-yl)glycinamide | 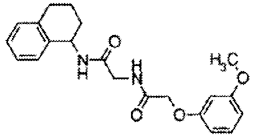 |

**Table 7**

| Entry | Name | Structure |
|---|---|---|
| 29 | 4-butyl-N-[2-oxo-2-(1,2,3,4-tetrahydronaphthalen-1-ylamino)benzamide | 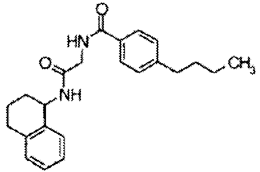 |
| 30 | N-2--{(2E)-3-[3,4-bis(methyloxy)phenyl]brob-2-enoyl}-N-(diphenylmethyl)-O-(phenylmethyl)serinamide | 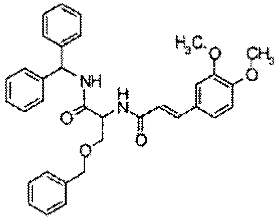 |
| 31 | N-(1,2,3,4-tetrahydronaphthalene-1-yl)-N~2~-[({4-[(trifluoromethyl)oxy]phenyl}ox y)acetyl]glycinamide | 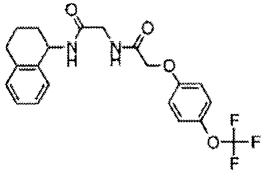 |
| 32 | N-{3-methyl-1-[(1,2,3,4-tetrahydronaphthalene-1-ylamino)carbonyl]butyl}bipheny l 4-carboxamide | 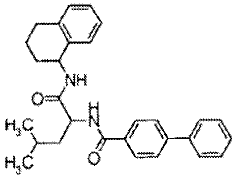 |
| 33 | N-2--({[4-(1,1-dimethylethyl)phenyl]oxy}acety 1)-N-(1,2,3,4-tetrahydronaphthalen-1-yl)glycinamide | 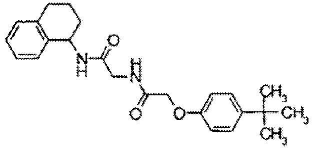 |

**Table 8**

| Entry | Name | Structure |
|---|---|---|
| 34 | N-2--{[(4-chlorophenyl)oxy]acetyl}-N-(1,2,3,4-tetrahydronaphthalen-1-yl)glycinamide | 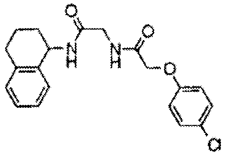 |
| 35 | N~2~-{[4-(pentyloxy)phenyl]carbonyl}-N-(1,2,3,4-tetrahydronaphthalene-1-yl)leucinamide | 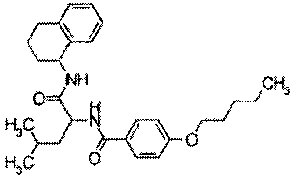 |
| 36 | 4-(hexyloxy)-N-[1-methyl-2-oxo-2-(1,2,3,4-tetrahydronaphthalen-2-ylamino)ethyl]benzamide | 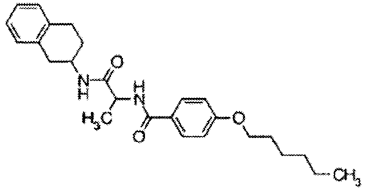 |
| 37 | 2-{[6-(methyloxy)-1,3-benzothiazol-2-yl]amino}-2-oxoethyl 3-phenyl-3-[(phenylcarbonyl)amino]propan oate | 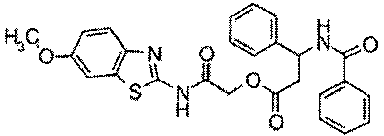 |
| 38 | N-[(3,4-dichlorophenyl)methyl]-2-{[4-(4-pyridin-2-ylpyrimidin-2-yl)phenyl]oxy}acetamide | 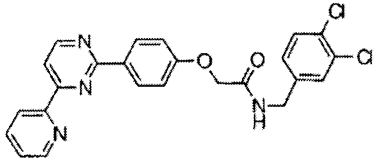 |

**Table 9**

| Entry | Name | Structure |
|---|---|---|
| 39 | N-(2,3-dihydro-1,4-benzodioxin-2-ylmethyl)-3-[3-(ethyloxy)propyl]-4-oxo-2-thioxo-1,2,3,4-tetrahydroquinazoline-7-carboxamide | 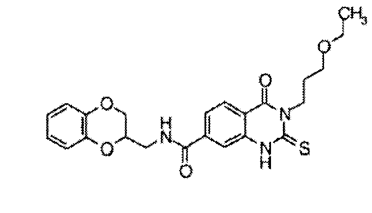 |
| 40 | N-2~-[(5,6-dimethyl-4-oxothieno[2,3-d]pyrimidin-3(4H)-yl)acetyl]-N-[6-(methyloxy)-1,3-benzothiazol-2-yl]glycinamide | 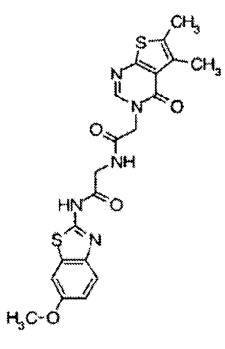 |
| 41 | 2-{4-[(2-naphthalene-1-ylethyl)sulfonyl]piperazin-1-yl}-N-pyridin-2-ylacetamide | 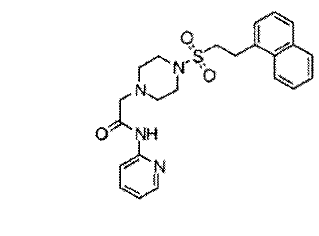 |
| 42 | N-(2,3-dihydro-1,4-benzodioxin-2-ylmethyl)-11-oxo-10,11-dihydro-6H-dibenzo[b,e][1,4]diazepine-8-carboxamide | 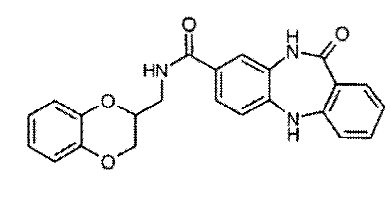 |

**Table 10**

| Entry | Name | Structure |
|---|---|---|
| 43 | N-[2-oxo-2-(1,2,3,4-tetrahydronaphthalen-1-ylamino)ethyl]biphenyl 4-carboxamide | 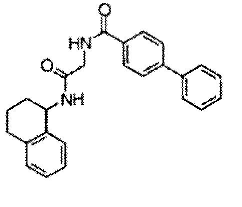 |
| 44 | N-[2-oxo-2-(1,2,3,4-tetrahydronaphthalene-1-ylamino)ethyl]naphthalene-2-carboxamide | 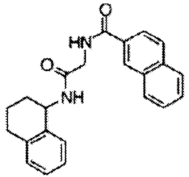 |
| 45 | N-[2-oxo-2-(1,2,3,4-tetrahydronaphthalen-1-ylamino)ethyl]-4-[(trifluoromethyl)oxy]benzamide | 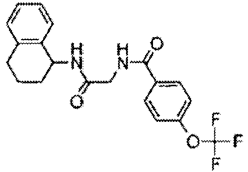 |
| 46 | N-[2-oxo-2-(1,2,3,4-tetrahydronaphthalene-1-ylamino)ethyl]quinoline-3-carboxamide | 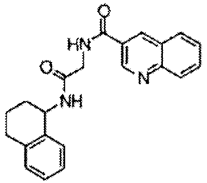 |

**Table 11**

| Entry | Name | Structure |
|---|---|---|
| 47 | N~2~-({[3,5-bis(trifluoromethyl)phenyl]amin o}carbonyl)-N-(1,2,3,4-tetrahydronaphthalen-1-yl)glycinamide | 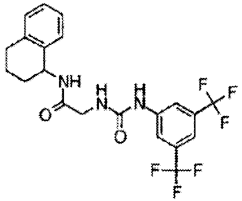 |
| 48 | N~2~-{[(3-ethylphenyl)amino]carbonyl}-N-(1,2,3,4-tetrahydronaphthalen-1-yl)glycinamide | 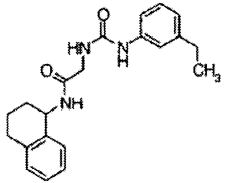 |
| 49 | N-[2-oxo-2-(1,2,3,4-tetrahydronaphthalen-1-ylamino)ethyl]-4-(phenyloxy)benzamide | 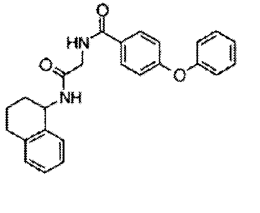 |
| 50 | 4-cyclohexyl-N-[2-oxo-2-(1,2,3,4-tetrahydronaphthalen-1-ylamino)ethyl]benzamide | 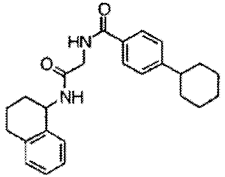 |

**Table 12**

| Entry | Name | Structure |
|---|---|---|
| 51 | N-[2-oxo-2-(1,2,3,4-tetrahydronaphthalen-1-ylamino)ethyl]-4-[(phenylmethyl)oxy]benzamid e | 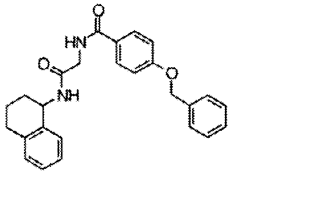 |
| 52 | 4'-ethyl-N-{2-oxo-2-[(phenylmethyl)amino]ethyl}bi phenyl 4-carboxamide | 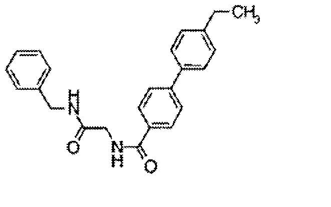 |
| 53 | N-[2-(cyclohexylamino)-2-oxoethyl]-4'-ethylbiphenyl 4-carboxamide | 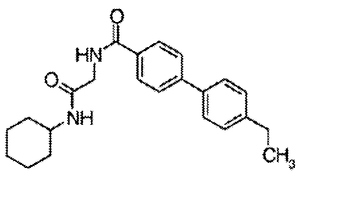 |
| 54 | N-(2-{[(3,4,-dichlorophenyl)methyl]amino}-2-oxoethyl)-4'-ethylbiphenyl 4-carboxamide | 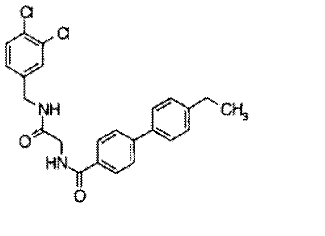 |
| 55 | N~2~-[{[cyclopentyl(phenyl)acetyl]a mino}(pyridin-3-yl)acetyl]-N-(1,2,3,4-tetrahydronaphthalen-1-yl)glycinamide | 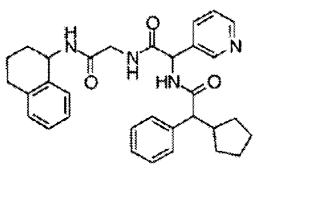 |

**Table 13**

| Entry | Name | Structure |
|---|---|---|
| 56 | 4-hydroxy-N-[2-oxo-2-(1,2,3,4-tetrahydronaphthalen-1-ylamino)ethyl]benzamide | 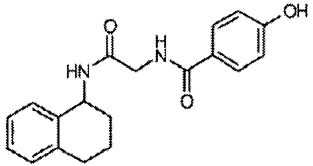 |
| 57 | N-[2-(1,3-dihydro-2H-isoindol-2-yl)-2-oxoethyl]-4'-ethylbiphenyl 4-carboxamide | 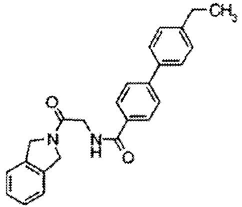 |
| 58 | 4-morpholin-4-yl-N-[2-oxo-2-(1,2,3,4-tetrahydronaphthalen-1-ylamino)ethyl]benzamide | 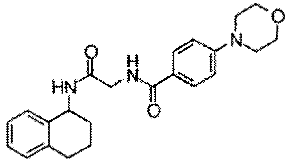 |
| 59 | 5,6-bis(methyloxy)-N-[2-oxo-2-(1,2,3,4-tetrahydronaphthalen-1-ylamino)ethyl]-1H-indole-2-carboxamide | 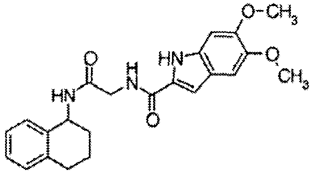 |
| 60 | N-[2-(3,4-dihydroisoquinolin-2(1H)-yl)-2-oxoethyl]-4'-ethylbiphenyl 4-carboxamide | 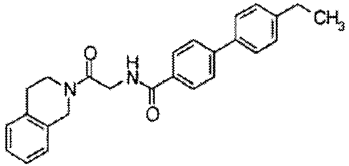 |

**Table 14**

| Entry | Name | Structure |
|---|---|---|
| 61 | 4'-ethyl-N-{2-oxo-2-[(1S)-1,2,3,4-tetrahydronaphthalen-1-ylamino]ethyl}biphenyl 4-carboxamide | 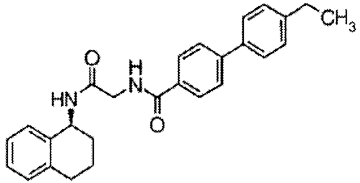 |
| 62 | 2-amino-N~4~-(2,3-dihydro-1,4-benzodioxin-2-ylmethyl)-N~1~-[3-(ethyloxy)propyl]benzene-1,4-dicarboxamide | 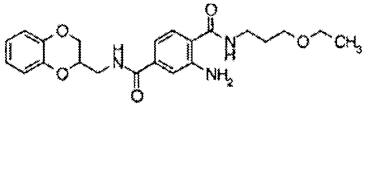 |
| 63 | N-{3-[(2-{[6-(methyloxine)-1, 3-benzothiazol-2-yl]amino}-2-oxoethyl)amino]-3-oxo-1-phenylpropyl}benzamide | 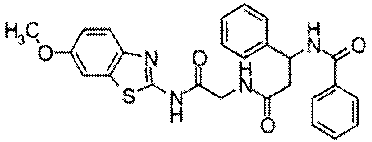 |
| 64 | 4'-ethyl-N-(2-{[6-(methyloxy)-1,3-benzothiazol-2-yl]amino}-2-oxoethyl)biphenyl 4-carboxamide | 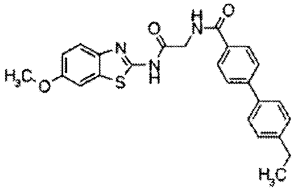 |
| 65 | N-{2-[(2,3-dihydro-1,4-benzodioxin-2-ylmethyl)amino]-2-oxoethyl}-4'-ethylbiphenyl 4-carboxamide | 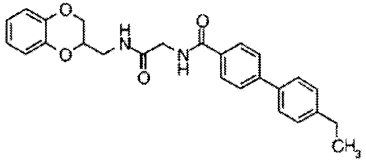 |

**Table 15**

| Entry | Name | Structure |
|---|---|---|
| 66 | 4'-ethyl-N-methyl-N-[2-oxo-2-(1,2,3,4-tetrahydronaphthalen-1-ylamino)ethyl]biphenyl 4-carboxamide | 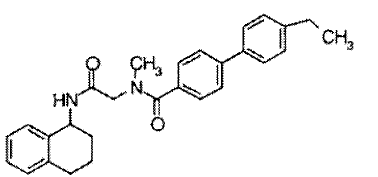 |
| 67 | 4'-ethyl-N-[2-(naphthalene-1-ylamino)-2-oxoethyl]biphenyl 4-carboxamide | 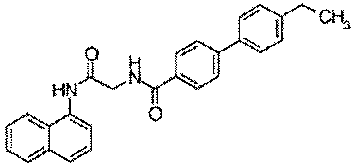 |
| 68 | 4'-ethyl-N-[2-oxo-2-(4-phenylpiperazin-1-yl)ethyl]biphenyl 4-carboxamide | 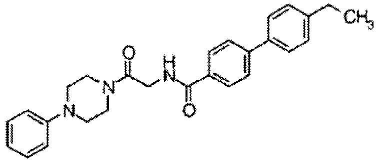 |
| 69 | N~2~-(biphenyl 4-ylmethyl)-N-(1,2,3,4-tetrahydronaphthalene-1-yl)glycinamide | 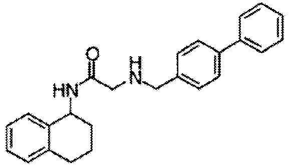 |
| 70 | 4-(1H-imidazol-1-yl)-N-[2-oxo-2-(1,2,3,4-tetrahydronaphthalen-1-ylamino)ethyl]benzamide | 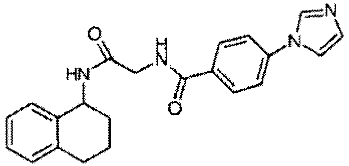 |

**Table 16**

| Entry | Name | Structure |
|---|---|---|
| 71 | 4-(1,3-oxazol-5-yl)-N-[2-oxo-2-(1,2,3,4-tetrahydronaphthalen-1-ylamino)ethyl]benzamide | 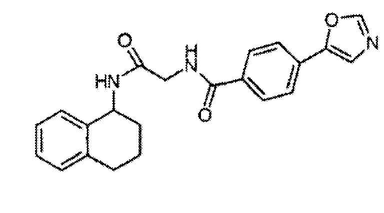 |
| 72 | N-2--{[(2-biphenyl 4-ylethyl)amino]carbonyl}-N-(1,2,3,4-tetrahydronaphthalen-1-yl)glycinamide | 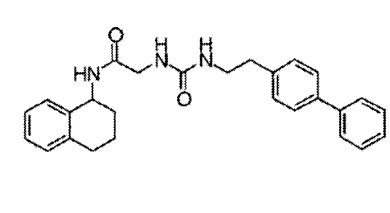 |
| 73 | N~2~-[(biphenyl 4-ylamino)carbonyl]-N-(1,2,3,4-tetrahydronaphthalene-1-yl)glycinamide | 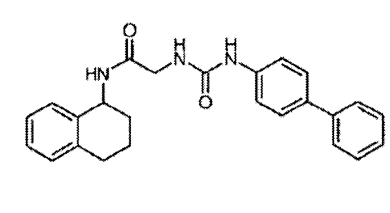 |
| 74 | 4'-ethyl-N-(2-{[(2-methylphenyl)methyl]amino}-2-oxoethyl)biphenyl 4-carboxamide | 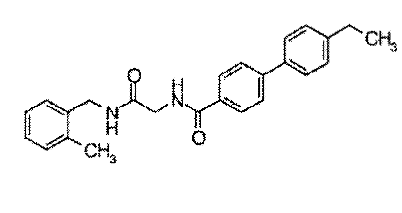 |
| 75 | 4'-ethyl-N-{2-oxo-2-[(2-phenylethyl)amino]ethyl}biphen yl 4-carboxamide | 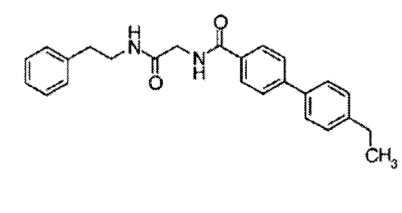 |

**Table 17**

| Entry | Name | Structure |
|---|---|---|
| 76 | 4'-ethyl-N-{2-oxo-2-[(1R)-1,2,3,4-tetrahydronaphthalen-1-ylamino]ethyl}biphenyl 4-carboxamide | 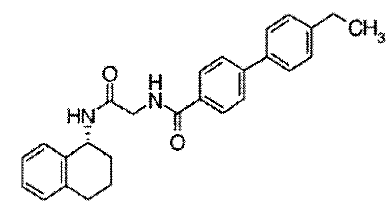 |
| 77 | N-[2-oxo-2-(1,2,3,4-tetrahydronaphthalen-1-ylamino)ethyl]-4-piperidin-1-ylbenzamide | 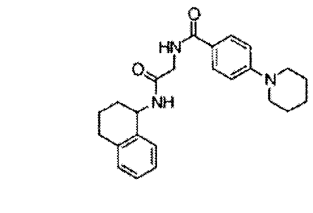 |
| 78 | 4'-ethyl-N-(2-oxo-2-[(1-phenylethyl)amino]ethyl}biphen yl 4-carboxamide | 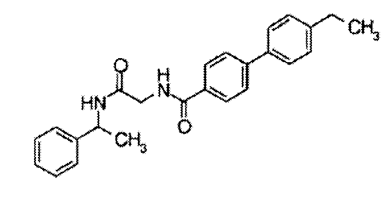 |
| 79 | 4'-ethyl-N-[2-oxo-2-({[2-(trifluoromethyl)phenyl]methyl}a mino)ethyl]biphenyl 4-carboxamide | 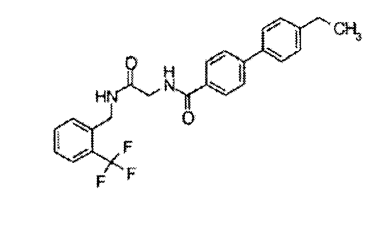 |
| 80 | N-[2-oxo-2-(1,2,3,4-tetrahydronaphthalen-1-ylamino)ethyl]quinoline-6-carboxamide | 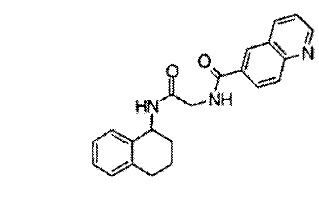 |

**Table 18**

| Entry | Name | Structure |
|---|---|---|
| 81 | N-(3-oxo-3-{[2-oxo-2-(1,2,3,4-tetrahydronaphthalen-1-ylamino)ethyl]amino}-1-phenylpropyl)benzamide | 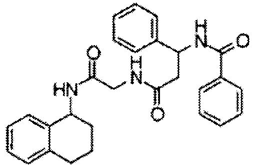 |
| 82 | N-[2-oxo-2-(1,2,3,4-tetrahydronaphthalene-1-ylamino)ethyl]-5,6,7,8-tetrahydronaphthalene-2-carboxamide | 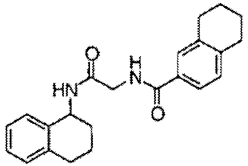 |
| 83 | 4'-ethyl-N-{2-oxo-2-[(1-phenylpropyl)amino]ethyl}biph enyl 4-carboxamide | 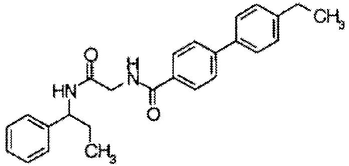 |
| 84 | 3-(acetylamino)-N-(2-{[6-(methyloxy)-1,3-benzothiazol-2-yl]amino}-2-oxoethyl)-3-phenylpropanamide | 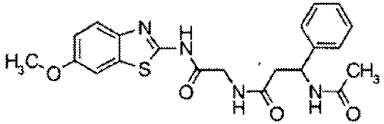 |
| 85 | N-(phenylcarbonyl)-β-alanyl-N-[6-(methyloxy)-1,3-benzothiazol-2-yl]glycinamide | 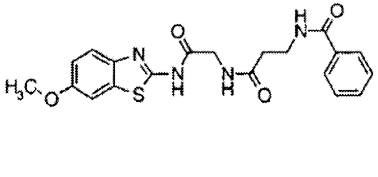 |

**Table 19**

| Entry | Name | Structure |
|---|---|---|
| 86 | N-[2-oxo-2-(5,6,7,8-tetrahydronaphthalen-1-ylamino)ethyl]quinoline-6-carboxamide | 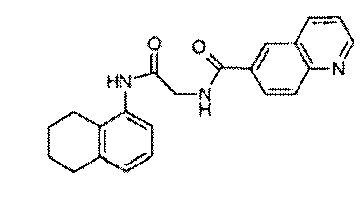 |
| 87 | N-[2-oxo-2-(5,6,7,8-tetrahydronaphthalen-1-ylamino)ethyl]quinoline-3-carboxamide | 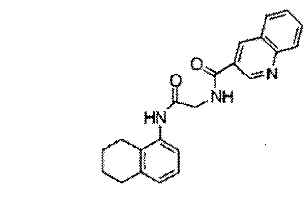 |
| 88 | N-[2-oxo-2-(1,2,3,4-tetrahydronaphthalen-1-ylamino)ethyl]-3-piperidin-1-ylpropanamide | 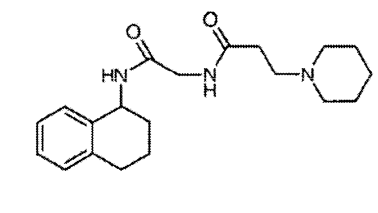 |
| 89 | N-[2-oxo-2-(5,6,7,8-tetrahydronaphthalen-1-ylamino)ethyl]-4-piperidin-1-ylbenzamide | 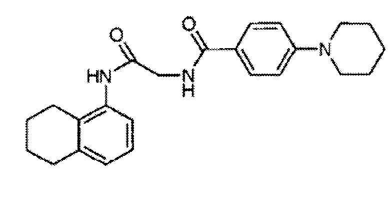 |
| 90 | N-(3-{[2-(1,3-benzothiazol-2-ylamino)-2-oxoethyl]amino}-3-oxo-1-phenylpropyl)benzamide | 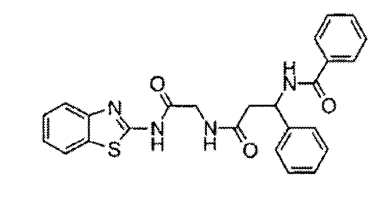 |

**Table 20**

| Entry | Name | Structure |
|---|---|---|
| 91 | N-[2-oxo-2-(1,2,3,4-tetrahydronaphthalen-1-ylamino)ethyl]-4-phenylpiperazine-1-carboxamide | 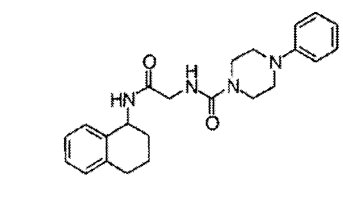 |
| 92 | N-(3-oxo-3-{[2-oxo-2-(1,3-thiazol-2-ylamino)ethyl]amino}-1-phenylpropyl)benzamide | 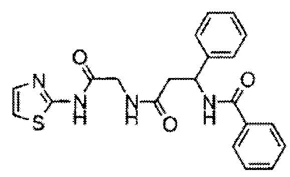 |
| 93 | N-[3-([2-[(4-methyl-1,3-thiazol-2-yl)amino]-2-oxoethyl]amino)-3-oxo-1-phenylpropyl]benzamide | 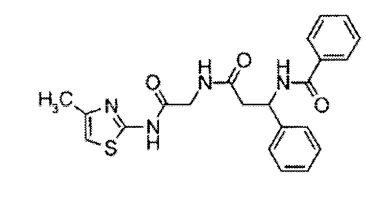 |
| 94 | N-{3-[(2-{[5-(methyloxy)[1,3]thiazolo[5,4-b]pyridin-2-yl]amino}-2-oxoethyl)amino]-3-oxo-1-phenylpropyl}benzamide | 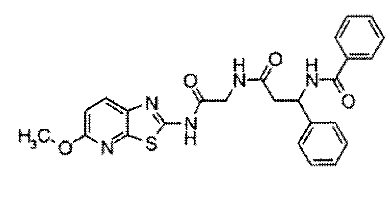 |

**Table 21**

| Entry | Name | Structure |
|---|---|---|
| 93 | 1,1-dimethylethyl 4-({N-[(4'-ethylbiphenyl 4-yl)carbonyl]glycyl}amino)-3,4-dihydroisoquinoline-2(1H)-carboxylate | 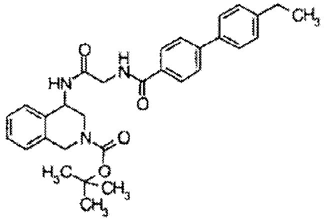 |
| 96 | 4'-ethyl-N-[2-oxo-2-(1,2,3,4-tetrahydroisoquinolin-4-ylamino)ethyl]biphenyl 4-carboxamide | 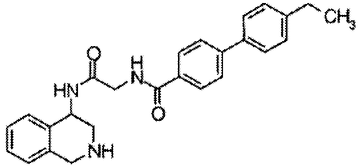 |
| 97 | N-[3-[(6-hydroxy-1,3-benzothiazol-2-yl)amino]-3-oxo-1-phenylpropyl)benzamide | 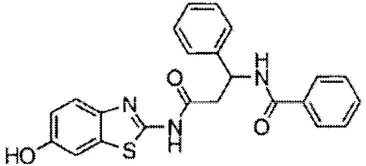 |
| 98 | N-[3-({2-[(6-hydroxy-1,3-benzothiazol-2-yl)amino]-2-oxoethyl}amino)-3-oxo-1-phenylpropyl]benzamide | 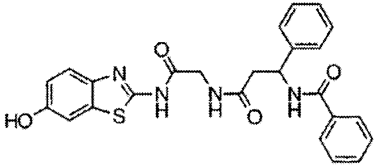 |

**Table 22**

| Entry | Name | Structure |
|---|---|---|
| 99 | 2-[(5-bromopyridin-2-yl)amino]-2-oxoethyl{[2-(naphthalen-1-ylamino)-2-oxoethyl]thio}acetate | 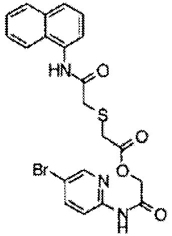 |
| 100 | 2-[(5-chloropyridin-2-yl)amino]-2-oxoethyl(1,3-benzoxazol-2-ylthio)acetate | 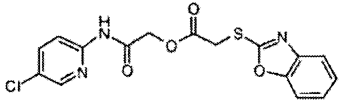 |
| 101 | 4-{[(5,6-dimethyl-4-oxothieno[2,3-d]pyrimidin-3(4H)-yl)acetyl]amino}-N-[6-(methyloxy)-1,3-benzothiazol-2-yl]butanamide | 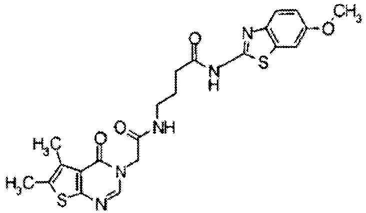 |

Examples of other TAOK1 function inhibitors include a TAOK1 antibody. The TAOK1 antibody is preferably an antibody having an antigen-binding property to an amino acid sequence near an active center of the serine-threonine protein kinase activity of TAOK1. By using such antibody, the TAOK1 function can be more reliably inhibited. A binding site can be determined on the basis of publicly known information and/or estimated on the basis of publicly known information (for example, by constructing a docking model or the like).

The antibody includes a polyclonal antibody, a monoclonal antibody, a chimeric antibody, a single-stranded antibody, or a portion of the above antibodies having an antigen-binding property as a Fab fragment, a fragment produced by a Fab expression library, or the like. Also included in the antibody of the present invention is an antibody having an antigen-binding property to a polypeptide containing at least 8 amino acids, preferably 15 amino acids, and more preferably 20 amino acids in succession of the amino acid sequence of TAOK1. These antibodies are available, and for example, ab67017 produced by Abcam Limited, STJ117738 produced by St Johns Laboratory Ltd, LS-C766558-60 produced by LifeSpan Biosciences, Inc. and the like have been known as an anti-TAOK1 antibody.

In addition, a method for producing these antibodies has been already well known, and the antibodies can be produced in accordance with these usual methods (Current protocols in Molecular Biology, Chapter 11.12-11.13 (2000)). Specifically, in a case where the antibody of the present invention is the polyclonal antibody, it is possible to use TAOK1 expressed and purified in E. coli or the like in accordance with a usual method, or synthesize an oligopeptide having a partial amino acid sequence of TAOK1 in accordance with a usual method to immunize a non-human animal such as a rabbit, thereby obtaining the antibody from the serum of the immunized animal in accordance with a usual method. Meanwhile, in a case of the monoclonal antibody, TAOK1 expressed and purified in E. coli or the like in accordance with the usual method, or the oligopeptide having the partial amino acid sequence of TAOK1 are used to immunize the non-human animal such as a mouse to obtain the antibody from hybridoma cells prepared by subjecting the obtained spleen cells and myeloma cells to cell fusion (Current protocols in Molecular Biology edit. Ausubel et al. (1987) Publish. John Wiley and Sons. Section 11.4-11.11).

TAOK1 used as an immune antigen in preparation of the antibody can be obtained by DNA cloning, construction of each plasmid, transfection into a host, culture of a transformant, and recovery of a protein from a culture based on publicly known gene sequence information. These operations can be performed in accordance with a method known to a person skilled in the art, a method described in a literature (Molecular Cloning, T. Maniatis et al., CSH Laboratory (1983), DNA Cloning, DM. Glover, IRL PRESS (1985)), or the like.

Specifically, a protein as the immune antigen for production of the antibody of the present invention can be obtained by preparing a recombinant DNA (expression vector) in which the gene encoding TAOK1 can be expressed in a desired host cell, introducing the recombinant DNA into the host cell to perform transformation, culturing the transformant, and recovering a target protein from the resulting culture. In addition, a partial peptide of TAOK1 can also be produced by a general chemical synthesis method (peptide synthesis) in accordance with publicly known gene sequence information.

In addition, the antibody of the present invention may also be prepared using the oligopeptide having the partial amino acid sequence of TAOK1. The oligo(poly)peptide used for the production of such antibody does not need to have functional biological activity, but desirably has an immunogenic property similar to that of TAOK1. Preferably, the oligo(poly)peptide can be taken as an example that has this immunogenic property and has at least 8 amino acids, preferably 15 amino acids, and more preferably 20 amino acids in succession of the amino acid sequence of TAOK1.

The production of the antibody against such oligo(poly)peptide can also be performed by enhancing an immunological reaction using various adjuvants depending on the host. Such adjuvants include, but are not limited to, a Freund's adjuvant, a mineral gel such as aluminum hydroxide, and a surface active substance such as lysolecithin, pluronic polyol, a polyanion, a peptide, an oil emulsion, a keyhole limpet hemocyanin, or dinitrophenol, and a human adjuvant such as BCG (Bacille Calmette-Guerin) or Corynebacterium parvum.

As the TAOK1 function inhibitor, in addition to the above, it is possible to use any molecule (for example, a peptide, a protein, an artificial antibody, or an aptamer) having a binding property (preferably, a specific binding property) to TAOK1. In addition, in a case where the protein or the peptide such as the antibody is employed as the TAOK1 function inhibitor, the expression cassette thereof may also be employed instead. The expression cassette is similarly defined as in "2-2-1. TAOK1 Expression Inhibitor" described above.

### 2-3. Applications and Other Components

As clarified in examples to be described later, the TAOK1 inhibitor has an inhibitory action on the myocardial cell death (for example, myocardial cell death due to mitochondrial dysfunction of the cardiomyocytes, a DNA damage of the cardiomyocytes, apoptosis of the cardiomyocytes, or a hypoxic load of the cardiomyocytes (for example, an ischemic heart disease such as myocardial infarction)) and an alleviating action on cardiac dysfunction (for example, cardiac dysfunction due to the myocardial cell death, the mitochondrial dysfunction of the cardiomyocytes, the DNA damage of the cardiomyocytes, the apoptosis of the cardiomyocytes, the hypoxic load of the cardiomyocytes (for example, the ischemic heart disease such as the myocardial infarction)). In addition, the TAOK1 inhibitor has the inhibitory action on the mitochondrial dysfunction (for example, a decrease in oxygen consumption, a decrease in mitochondrial membrane potential, and an increase in reactive oxygen species) of the cardiomyocytes, the inhibitory action on the DNA damage of the cardiomyocytes, and the inhibitory action on the apoptosis of the cardiomyocytes.

In particular, the TAOK1 inhibitor has an inhibitory action on the myocardial cell death caused by the anticancer agent treatment, and further has an alleviating action on the cardiac dysfunction caused by the anticancer agent treatment. In particular, the TAOK1 inhibitor has an inhibitory action on the mitochondrial dysfunction (for example, the decrease in oxygen consumption, the decrease in mitochondrial membrane potential, and the increase in reactive oxygen species) of the cardiomyocytes caused by the anticancer agent treatment, an inhibitory action on the DNA damage of the cardiomyocytes caused by the anticancer agent treatment, and an inhibitory action on the apoptosis of the cardiomyocytes caused by the anticancer agent treatment.

At least one (active component) selected from the group consisting of the TAOK1 expression inhibitor and the TAOK1 function inhibitor is effective for inhibiting the myocardial cell death (in particular, the myocardial cell death caused by the anticancer agent treatment or the myocardial cell death caused by the hypoxic load of the cardiomyocytes (for example, the ischemic heart disease such as the myocardial infarction)), preventing or treating myocardial disorder/heart failure (in particular, a myocardial disorder/heart failure caused by the anticancer agent treatment or a myocardial disorder/heart failure caused by the hypoxic load of the cardiomyocytes (for example, the ischemic heart disease such as the myocardial infarction)), inhibiting the mitochondrial dysfunction (for example, the decrease in oxygen consumption, the decrease in mitochondrial membrane potential, and the increase in reactive oxygen species) of the cardiomyocytes (in particular, the mitochondrial dysfunction of the cardiomyocytes caused by the anticancer agent treatment), inhibiting the DNA damage of the cardiomyocytes (in particular, the DNA damage of the cardiomyocytes caused by the anticancer agent treatment), and inhibiting the apoptosis of the cardiomyocytes (in particular, the apoptosis of the cardiomyocytes caused by the anticancer agent treatment). The active component can be utilized as an active component of, for example, a food composition, a health promoting agent, or a nutritional auxiliary agent (such as a supplement), in addition to a medicine and a reagent. The active component can be unchangeably used or can be used as various compositions together with a conventional component, and can be applied (for example, administered, ingested, or inoculated) to animals, humans, and various cells.

The anticancer agent is not particularly limited as long as the agent causes the myocardial cell death, the cardiac dysfunction, the mitochondrial dysfunction of the cardiomyocytes, the DNA damage of the cardiomyocytes, or the apoptosis of the cardiomyocytes. Suitable examples include an anthracycline-based anticancer agent. Examples of the anthracycline-based anticancer agent include the doxorubicin, daunorubicin, epirubicin, liposomal doxorubicin, and mitoxantrone. Other examples of the anticancer agent include an anti-HER2 antibody (trastuzumab), a VEGF inhibitor (bevacizumab), a tyrosine kinase inhibitor (sunitinib, imatinib, lapatinib, or osimertinib), and an MEK inhibitor (trametinib).

The agent of the present invention is preferably used to be administered in combination with the anticancer agent. This makes it possible to inhibit, prevent, and treat the myocardial cell death, the myocardial disorder, the heart failure, the mitochondrial dysfunction of the cardiomyocytes, the DNA damage of the cardiomyocytes, the apoptosis of the cardiomyocytes, and the like caused by the anticancer agent treatment. The timing of the combination use is not particularly limited, and for example, the agent of the present invention and the anticancer agent may be ingested simultaneously or on the same day, or the former may be ingested one or more days (for example, 2 to 14 days) before or after the ingestion date of the latter.

The cell type of the cell targeted by the agent of the present invention is not particularly limited as long as the target cell can express TAOK1, and the target cell is particularly preferably the myocardial cell.

The organism targeted by the agent of the present invention is not particularly limited, and examples thereof include various mammalian animals such as humans, monkeys, mice, rats, dogs, cats, rabbits, pigs, horses, cows, sheep, goats, and deer.

The form of the agent of the present invention is not particularly limited, and may be a form that is usually used in each application depending on the application of the agent of the present invention.

In a case where the agent applied to a medicine, a health promoting agent, a nutritional auxiliary agent (such as a supplement) or the like, examples of the form include formulations suitable for oral intake (oral formulations) such as tablets (including orally disintegrating tablets, chewable tablets, foamed tablets, lozenges, jelly-like drops, or the like), pills, granules, fine granules, powders, hard capsules, soft capsules, dry syrups, solutions (including drinks, suspensions, and syrups), and jellies, and formulations (parenteral formulations) suitable for parenteral intake such as nasal drops, inhalants, rectal suppositories, intercalating agents, enteral enemas, jellies, injections, patches, lotions, and creams.

In a case where the agent is applied to a food composition, examples of the form include liquid, gelatinous, or solid food such as juice, soft drink, tea, soup, soy milk, salad oil, dressing, yogurt, jelly, pudding, sprinkling, powdered milk for nursing, cake mix, powdered or liquid dairy products, bread, and cookie.

The agent of the present invention may further contain other components as necessary. The other components are not particularly limited as long as the components can be blended in, for example, a medicine, a food composition, a health promoting agent, or a nutritional auxiliary agent (such as a supplement), and examples thereof include a base, a carrier, a solvent, a dispersant, an emulsifier, a buffer, a stabilizer, an excipient, a binder, a disintegrator, a lubricant, a thickener, a humectant, a colorant, a fragrance, and a chelating agent.

The content of the active component in the agent of the present invention depends on the type of the active component, the application, the use mode, the application target, the state of the application target, and the like, and can be, for example, 0.0001 to 100 wt%, and preferably 0.001 to 50 wt%, and is not limited thereto.

The amount of the agent of the present invention to be applied (for example, administered, ingested, or inoculated) is not particularly limited as long as the amount is effective for exhibiting drug efficacy, and the weight of the active component is generally 0.1 to 1000 mg/kg of a body weight per day. The above amount is preferably administered once or 2 to 3 times a day, and can also be appropriately increased or decreased depending on an age, a disease state, and a symptom.

### 3. Screening Method

In one aspect, the present invention relates to the method (in the present description, also referred to as an "active component screening method of the present invention") for screening the active component of the inhibitory agent for the myocardial cell death or the active component of the prophylactic or therapeutic agent for at least one heart disease selected from the group consisting of the myocardial disorder and the heart failure, using, as the index, the amount, the concentration, or the kinase activity of TAOK1 in the test sample derived from the animal or the cell treated with the test substance. This will be described below.

The test sample is not particularly limited as long as the sample can detect TAOK1 derived from an animal or a cell. Examples of the test sample preferably include the cardiomyocytes.

The method for measuring the amount and the concentration of TAOK1 is not particularly limited. Examples of the method include a mass spectrometry method, an immunological measurement method (for example, an ELISA method, an EIA method, an RIA method, or a western blotting method) using an antibody that specifically recognizes a target biomarker, a northern hybridization method, a DNA microarray method, and a PCR method.

The method for measuring the kinase activity of TAOK1 is also not particularly limited. The kinase activity can be measured according to or in accordance with a publicly known kinase activity measurement method.

The biological species of the animal is not particularly limited. Examples of the biological species of the animal include various mammalian animals such as monkeys, mice, rats, dogs, cats, and rabbits.

As the test substance, various substances can be used regardless of a naturally occurring compound or an artificially produced compound. In addition, not only a purified compound but also a composition obtained by mixing various types of compounds and an extract of a plant or an animal can also be used. The compound is not limited to the low molecular weight compound, and also includes a high molecular weight compound such as a protein, a nucleic acid, or a polysaccharide.

More specifically, the active component screening method of the present invention includes a step of selecting the test substance as the active component of the inhibitory agent of the myocardial cell death or the active component of the prophylactic or therapeutic agent for at least one heart disease selected from the group consisting of the myocardial disorder and the heart failure, in a case where values of the indexes related to TAOK1 are lower than the values of the indexes related to TAOK1 in the test sample without being treated with the test substance.

"Lower" means, for example, that the values of the indexes are 9/10, 8/10, 7/10, 6/10, 1/2, 1/5, 1/10, 1/20, 1/50, or 1/100 or less of control values.

### Examples

Hereinafter, the present invention will be described in detail based on examples, and the present invention is not limited by these examples.

### Test Example 1. Screening Using Cardiomyocytes

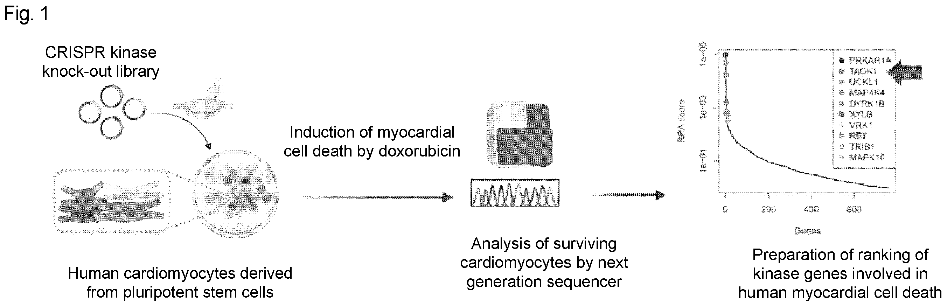

Genes involved in the myocardial cell death by the doxorubicin were screened in accordance with a scheme illustrated in Fig. 1. Specifically, the screening was performed as follows.

### 1-1. Cell Culture

Human pluripotent stem cells derived from a healthy human (iPS cells) (SCVI-273) were supplied by Stanford University Cardiovascular Institute Biobank (https://med.stanford.edu/scvibiobank.html), and subjected to maintenance culture in an Essential 8 medium (Thermo Fisher) in a non-feeder environment on a growth factor reduced matrigel (Corning). Directed differentiation was performed on the cardiomyocytes using the low molecular weight compound based on a previous report (Circulation. 2019 May 21;139(21):2451-2465. doi: 10.1161/CIRCULATIONAHA.118.037357), purified in a glucose free medium, and then used for an experiment in which maintenance was performed in RPMI1640 and B27 media (Thermo Fisher). The iPS cells and iPS cell-derived cardiomyocytes were detached with Accutase (Nacalai Tesque) and Accutase (Nacalai Tesque), respectively. All cell lines were maintained in an incubator under an environment of 5% CO₂ and 37 degrees Celsius.

### 1-2. Creation of Lentivirus-Based CRISPR Single Vector Construct

A lentiviral vector was produced from a Cas9-expressing lentiviral library (addgene plasmid #75314, #75315) targeting a target gRNA for all 763 kinases using a packaging system (using Lenti-X 293T as a packaging cell line) using psPAX2 (addgene plasmid # 12260) and pMD2.G (addgene plasmid #12259), and recovered as a viral solution after 48 to 72 hours.

### 1-3. Survival Myocardial Cell Screening Using CRISPR/Cas9 System

With a Cas9-expressing lentiviral gRNA library, 5 million to10 million iPS cell-derived cardiomyocytes were infected at MOI = 0.3-0.5, and uninfected cells were killed with puromycin (2 µg/ml). Thereafter, 14 days later, each of a treated group for 72 hours with the doxorubicin (0.5 µM) and an untreated group was prepared, and the cells were recovered. Genomic DNAs were recovered from the recovered cells using NucleoSpin (registered trademark) Tissue (Macherey-Nagel), and the library was adjusted by 3-step-PCR based on a previous report (Nature. 2019 Nov;575(7782):375-379. doi: 10.1038/s41586-019-1667-4). The adjusted library was sequenced using NovaSeq 6000, and after adaptor sequences were removed by Cutadapt, alignment was performed on the basis of Brunello library gRNA sequence information, and RRA scores of each of the genes were calculated using a MAGeCK program (Genome Biol. 2014;15(12):554. doi: 10.1186/s13059-014-0554-4).

### 1-4. Results

TAOK1 was selected as a candidate gene from the top ranking of the RPA scores. TAOK1 has been known to have an effect on cancer (PTL 1), but is not known to be associated with the myocardial cell death.

### Test Example 2. Evaluation 1 of TAOK1 Gene as Target Gene

TAOK1 was knocked out and the viability of the cardiomyocytes after the doxorubicin treatment was examined. Specifically, the procedures were performed as follows. Unless otherwise specified below, similar procedures were performed as in Test Example 1.

### 2-1. TAOK1 Knock-out Using CRISPR/Cas9 System

Two types of gRNAs targeting TAOK1 (TAOK 1-1, 1-2) and two types of non-target gRNAs (NTC 1, 2) were incorporated into LentiCRISPRv2puro (addgene plasmid #98290), the lentiviral vector was produced in a similar manner as described above, the iPS cell-derived cardiomyocytes were infected with the lentiviral vector at MOI = 0.8-1.0, and uninfected cells were killed with the puromycin (2 µg/ml). Thereafter, the iPS cell-derived cardiomyocytes were re-seeded at a cell concentration of 120,000 cells/cm2, and used for the experiment after 14 days or more.

### 2-2. Quantification of Cell Viability

After the infection with the lentiviral vector, the iPS cell-derived cardiomyocytes subjected to drug selection with the puromycin were seeded on a 96-well plate coated with the growth factor reduced matrigel. The doxorubicin was added after 14 days or more from the infection with the viral vector, and after 3 days, the cell viability was measured with SpectraMaxM2e (molecular devices) using a PrestoBlue reagent (Thermo Fisher).

### 2-3. Live Cell/Dead Cell Staining

After the infection with the lentiviral vector, the iPS cell-derived cardiomyocytes subjected to the drug selection with the puromycin were seeded on a glass bottom dish coated with the growth factor reduced matrigel, and the doxorubicin was added after 14 days or more from the infection with the viral vector. After one day, staining was performed with a Cellstain (registered trademark) Double Staining Kit (Dojindo), and imaging was performed with BZ-X800 (Keyence).

### 2-4. Results

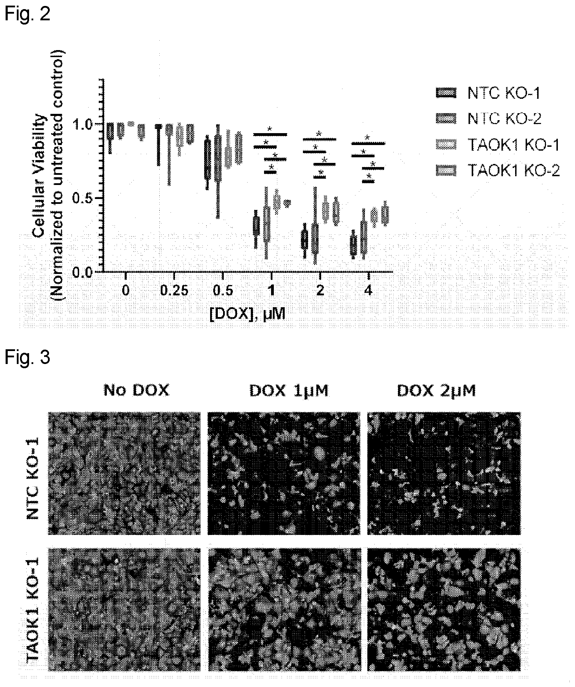

Quantitative results of the cell viability are illustrated in Fig. 2. In addition, the results of the live cell/dead cell staining are illustrated in Fig. 3. The TAOK1 knock-out was found to significantly improve the viability of the cardiomyocytes after the doxorubicin treatment. It has been known that the myocardial cell death is involved in onset and deterioration of heart diseases, particularly the myocardial disorders and the heart failure. Therefore, TAOK1 inhibition was considered to be effective for the prevention or treatment of these diseases.

### Test Example 3. Evaluation 2 of TAOK1 Gene as Target Gene

The viability of the cardiomyocytes was examined after the treatment with the TAOK1 inhibitor (PTL 1: the compound 43) and the doxorubicin treatment. Specifically, the procedures were performed as follows. Unless otherwise specified below, similar procedures were performed as in Test Example 1.

The iPS cell-derived cardiomyocytes were seeded on the 96-well plate coated with the growth factor reduced matrigel, after 5 days or more from the seeding, the TAOK1 inhibitor C43 (MedChemExpress) and the doxorubicin were simultaneously added, and 3 days later, the cell viability was measured with SpectraMaxM2e using the PrestoBlue reagent.

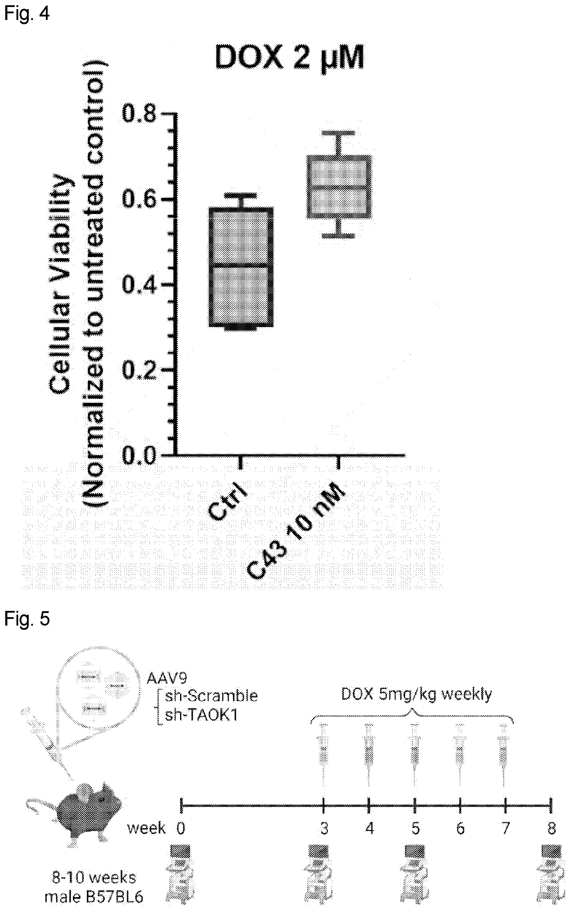

The results are illustrated in Fig. 4. The TAOK1 inhibitor was found to inhibit the myocardial cell death caused by the doxorubicin.

### Test Example 4. Evaluation 3 of TAOK1 Gene as Target Gene

TAOK1 was knocked down, and cardiac function evaluation and gene expression measurement after the doxorubicin treatment were performed. The outline is illustrated in Fig. 5. Specifically, the procedures were performed as follows.

A hairpin RNA (shRNA)-expressing self-complementary adeno-associated virus serotype 9 vector (AAV9-sh) was produced by VectorBuilder Inc. The vector was administered to each of wild-type C57BL/6 male mice at an age of 8 to 10 weeks through 1 × 10¹¹ vg orbital plexus using AAV9-sh-TAOK1 targeting TAOK1 or non-target AAV-sh-Scramble, and after 3 weeks, the doxorubicin was administered intraperitoneally to mice at 5 mg/kg every week for 5 weeks, and the cardiac functions were evaluated using a cardiac ultrasound device Vevo 2100 (FUJIFILM). The mice after being subjected to the experiment were euthanized by administration of a large amount of an anesthetic, and the hearts thereof were recovered. The recovered mouse hearts were subjected to total RNA extraction using a Direct-zol RNA Kit (Zymo Research). Reverse transcription was performed from the recovered RNA using a PrimeScript RT reagent Kit (Takara), and gene expressions were evaluated using a KAPA SYBR FAST qPCR kit in a CFX 384 Real-Time system (Biorad).

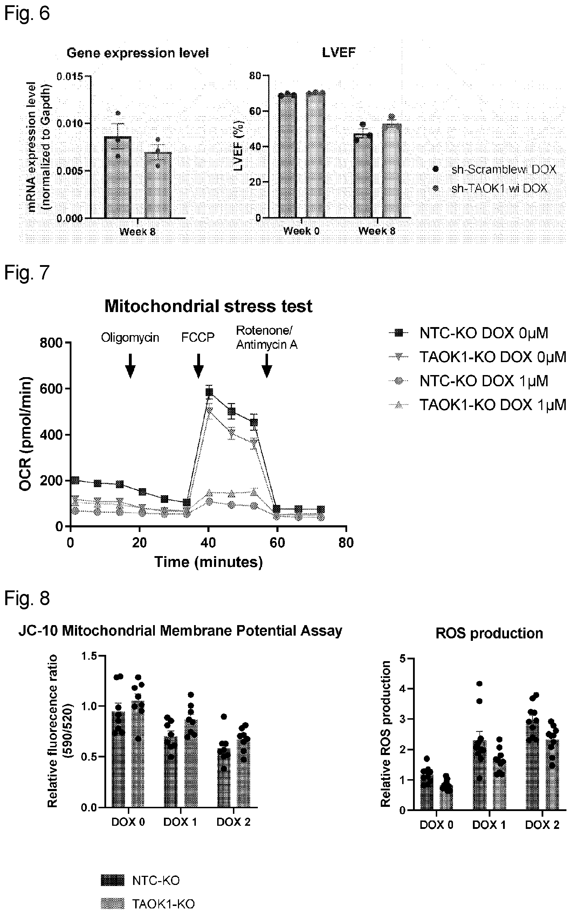

The results are illustrated in Fig. 6. The TAO1 knockdown was found to alleviate the cardiac dysfunction by the doxorubicin.

### Test Example 5. Evaluation 4 of TAOK1 Gene as Target Gene

TAOK1 knock-out cells were obtained from the iPS cell-derived cardiomyocytes in a similar manner as in Test Example 2-1. The obtained cells were treated with the doxorubicin at 0 µM or 1 µM for 3 hours, and oxygen consumption was measured using a Seahorse XFe 96 Flux Analyzer (Agilent) and a Seahorse XF Cell Mito Stress Test Kit (Agilent).

The measurement results of the oxygen consumption are illustrated in Fig. 7. The doxorubicin treatment significantly reduced the oxygen consumption of the cardiomyocytes. The decrease in oxygen consumption was alleviated in a TAOK1 knock-out group as compared with a non-specific knock-out group.

### Test Example 6. Evaluation 5 of TAOK1 Gene as Target Gene

The TAOK1 knock-out cells were obtained from the iPS cell-derived cardiomyocytes in a similar manner as in Test Example 2-1. The obtained cells were treated with the doxorubicin at 0, 1, or 2 µM for 3 hours, and the mitochondrial membrane potentials and reactive oxygen species (ROS) were measured using a Cell Meter JC-10 (AAT bioquest) and ROS Assay (Dojindo), respectively.

The measurement results of the mitochondrial membrane potentials and production amounts of the reactive oxygen species are illustrated in Fig. 8. The decrease in mitochondrial membrane potential and generation of the reactive oxygen species due to the doxorubicin treatment were alleviated by the TAOK1 knock-out.

### Test Example 7. Evaluation 6 of TAOK1 Gene as Target Gene

The TAOK1 knock-out cells were obtained from the iPS cell-derived cardiomyocytes in a similar manner as in Test Example 2-1. The obtained cells were treated with the doxorubicin at 0 or 1 µM for 24 hours, and the cells were recovered with Accutase (Nacalai Tesque). After fixation with 4% PFA, the staining was performed with a Purified anti-H2A.X Phospho (Ser139) antibody (biolegend, 613401) and an Anti-Cardiac Troponin T antibody (abcam, ab45932), and the γH2AX-positive cells in the troponin T-positive cells were detected by flow cytometry (SONY, MA900).

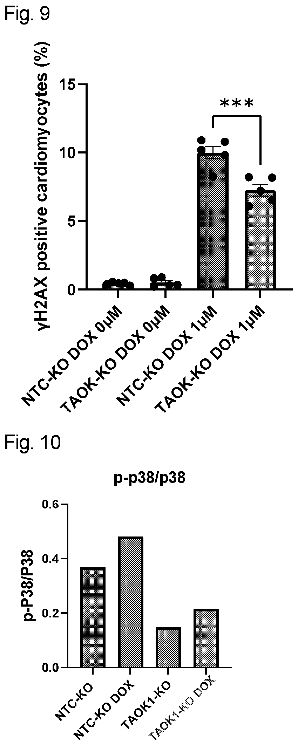

The proportion of the γH2AX-positive cells in the troponin T-positive cells is illustrated in Fig. 9. An increase in γH2AX-positive cells, which are DNA damage markers, due to the doxorubicin treatment was alleviated due to the TAOK1 knock-out.

### Test Example 8. Evaluation 7 of TAOK1 Gene as Target Gene

The TAOK1 knock-out cells were obtained from the iPS cell-derived cardiomyocytes in a similar manner as in Test Example 2-1. The obtained cells were treated with the doxorubicin at 1 µM for 24 hours to recover proteins, and phosphorylation of the p38 was evaluated by western blot (9216, 9212 Cell Signaling Technology) .

The measurement results of the proportion of the amount of the phosphorylated p38 to the amount of the p38 are illustrated in Fig. 10. In TAOK1 knock-out cardiomyocytes, suppression of a p38 pathway was observed, and activation of the p38 due to the doxorubicin was inhibited.

### Test Example 9. Evaluation 8 of TAOK1 Gene as Target Gene

Using cardiomyocytes induced to differentiate from human iPS cells (line1: SCVI-273 and line2: SCVI-116), Hela cells derived from human cervical cancer, and MCF7 cells derived from human breast cancer, knockdown of the TAOK1 gene was performed with a siRNA (Silencer Select, Thermo Fisher Scientific), and the doxorubicin at 2 µM was administered from day 3 of siRNA administration. On day 3 after the administration of the doxorubicin, the cell viability was measured with the PrestoBlue reagent.

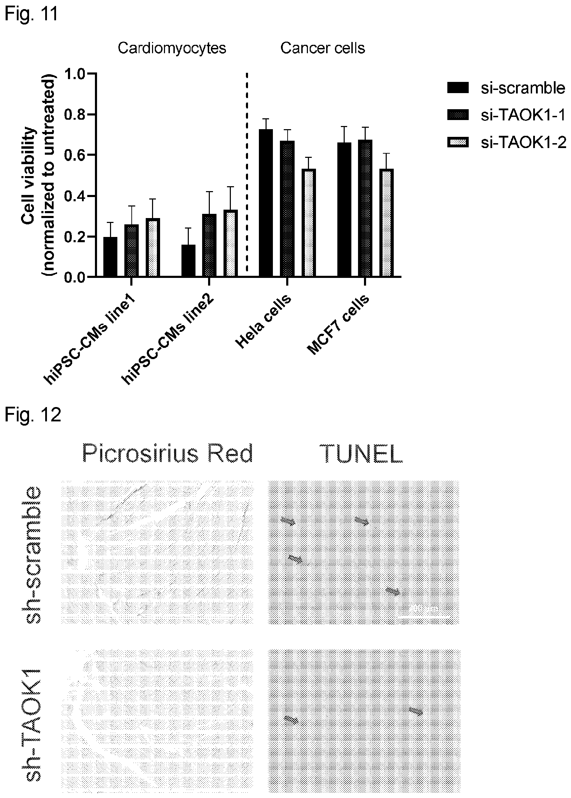

The measurement results of the cell viability are illustrated in Fig. 11. The doxorubicin treatment improved the cell viability of a TAOK1-knockdown group as compared with a non-specific gene knockdown group in human cardiomyocytes. Meanwhile, in a human cancer cell line, an improvement in cell viability of the TAOK1 knockdown group was not observed as compared with the non-specific gene knockdown group.

### Test Example 10. Evaluation 9 of TAOK1 Gene as Target Gene

The mouse hearts recovered during the evaluation of the cardiac functions in Test Example 4 (Week 8 in Fig. 6) were used. The recovered hearts were fixed with 4% PFA, embedded in paraffin, and made into sectioned slides, and fibrosis was evaluated by Picosirius Red and the apoptosis was evaluated by TUNEL staining.

The tissue staining images are illustrated in Fig. 12. The fibrosis and the cell death in the mouse hearts due to the doxorubicin were alleviated in the TAOK1 knockdown group as compared with the non-specific gene knockdown group.

### Test Example 11. Evaluation 10 of TAOK1 Gene as Target Gene

The TAOK1 gene was knocked down by the siRNA (Silencer Select, Thermo Fisher Scientific) in human iPS cell-derived cardiomyocytes, and cultured for 16 hours under 0.1% oxygen conditions with a BIONIX hypoxic culture kit (Sugiyamagen) from day 3 of the siRNA administration to induce the cell death. The live cells were stained by a Cellstain-Double Staining Kit (Dojindo), followed by image acquisition.

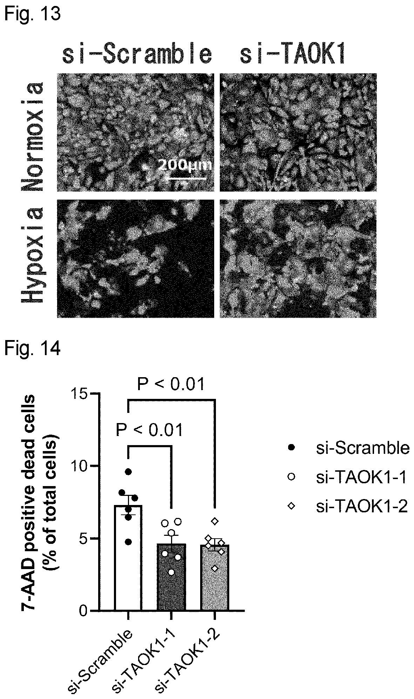

The cell staining images are illustrated in Fig. 13. In the TAOK1 knockdown group, the cardiomyocytes that survive under the hypoxic conditions increased.

### Test Example 12. Evaluation 11 of TAOK1 Gene as Target Gene

The TAOK1 gene was knocked down by the siRNA (Silencer Select, Thermo Fisher Scientific) in the human iPS cell-derived cardiomyocytes, and cultured for 16 hours under 0.1% oxygen conditions with the BIONIX hypoxic culture kit (Sugiyamagen) from day 3 of the siRNA administration to induce the cell death. The dead cells were quantitatively evaluated by the flow cytometry using a 7AAD reagent.

The proportion of the dead cells is illustrated in Fig. 14. In the TAOK1 knockdown group, the myocardial cell death was reduced under the hypoxic conditions.

## Claims

1. An inhibitory agent for myocardial cell death, comprising at least one component selected from the group consisting of a TAOK1 function inhibitor and a TAOK1 expression inhibitor.

2. The inhibitory agent according to Claim 1, wherein the component is at least one selected from the group consisting of a polynucleotide targeting TAOK1, an expression cassette of the polynucleotide, a low molecular weight compound, a peptide, a protein, and an antibody.

3. The inhibitory agent according to Claim 1, wherein the myocardial cell death is myocardial cell death caused by an anticancer agent treatment.

4. The inhibitory agent according to Claim 1, used to be administered in combination with an anticancer agent.

5. The inhibitory agent according to Claim 3 or 4, wherein the anticancer agent is an anthracycline-based anticancer agent.

6. The inhibitory agent according to Claim 1, for use in prevention or treatment of a heart disease.

7. The inhibitory agent according to Claim 6, wherein the heart disease is at least one heart disease selected from the group consisting of a myocardial disorder and heart failure.

8. The inhibitory agent according to Claim 6, wherein the heart disease is a heart disease caused by an anticancer agent treatment.

9. A prophylactic or therapeutic agent for at least one heart disease selected from the group consisting of a myocardial disorder and heart failure, the prophylactic or therapeutic agent containing at least one component selected from the group consisting of a TAOK1 function inhibitor and a TAOK1 expression inhibitor.

10. The prophylactic or therapeutic agent according to Claim 9, wherein the heart disease is a heart disease caused by an anticancer agent treatment.

11. A method for screening an active component of an inhibitory agent for myocardial cell death or an active component of a prophylactic or therapeutic agent for at least one heart disease selected from the group consisting of a myocardial disorder and heart failure, using, as an index, an amount, a concentration, or kinase activity of TAOK1 in a test sample derived from an animal or a cell treated with a test substance.
